# EUROPEAN PATENT APPLICATION

(11) **EP 3 696 284 A1**
(43) Date of publication of application: **19.08.2020**
(21) Application number: 18866818.0
(22) Date of filing: 13.09.2018
(51) Int. Cl.: C12Q 1/6895

(54) **METHOD FOR DIAGNOSING ALZHEIMER DEMENTIA VIA BACTERIAL METAGENOMIC ANALYSIS**

(30) Priority: 13.10.2017 KR 20170133372; 28.05.2018 KR 20180060753
(71) Applicant: MD Healthcare Inc., Seoul 03923 (KR); Ewha University-Industry Collaboration Foundation, Seoul 03760 (KR)
(72) Inventor: KIM, Yoon-Keun, Paju-si Gyeonggi-do 10908 (KR); HAN, Pyung Lim, Seoul 07671 (KR); PARK, Jin-young, Daejeon 34076 (KR)
(74) Representative: Dehns
(86) International application number: PCT/KR2018/010776
(87) International publication number: WO 2019/074216

(57) **Abstract**

The present invention relates to a method of diagnosing Alzheimer's dementia by bacterial metagenomic analysis, and more particularly, to a method of diagnosing Alzheimer's dementia and mild cognitive impairment by analyzing an increase or decrease in contents of specific bacterial extracellular vesicles by performing bacterial metagenomic analysis using normal person and subject-derived samples. The extracellular vesicles secreted from bacteria present in the environment are absorbed into the body and penetrate into brain tissue to have a direct influence on a cognitive function such as Alzheimer's dementia, and since Alzheimer's dementia is difficult to be diagnosed early before symptoms are shown, effective treatment is difficult. Therefore, by previously predicting the risk of the onset of mild cognitive impairment and Alzheimer's dementia through metagenomic analysis of bacterial extracellular vesicles using a human-derived sample according to the present invention, an Alzheimer's dementia risk group may be diagnosed and predicted early to delay the time of onset or prevent the onset of Alzheimer's dementia with proper cure, and after the onset of the disease, early diagnosis may be performed, thereby reducing the incidence of Alzheimer's dementia and increasing a therapeutic effect.

## Description

### [Technical Field]

The present invention relates to a method for diagnosing Alzheimer's dementia through a bacterial metagenomic analysis and, more specifically, to a method of diagnosing Alzheimer's dementia, and the like by performing a bacterial metagenomic analysis using normal individual-derived and subject-derived samples to analyze an increase or decrease in the content of specific bacteria-derived extracellular vesicles.

### [Background Art]

Dementia is an overall term for diseases that cause a gradual decrease in major brain functions and memory. Alzheimer's dementia is the most common type of dementia, and 75% of dementia patients have Alzheimer's dementia. Alzheimer's dementia is found in 10% of people 65 years or older and 30 to 50% people of 85 years or older.

The cause of Alzheimer's dementia is not clearly understood, but according to its pathological mechanism, Alzheimer's dementia is defined as a degenerative nervous system disease which has neuropathological symptoms such as the excessive deposition of β-amyloid plaques outside neurons and the excessive production of a hyperphosphorylated tau protein in neurons, resulting in gradual cognitive dysfunction due to neuronal function impairment such as neuroplasticity and neuronal death. The causes of Alzheimer's dementia are divided into genetic and environmental causes. As the genetic causes, variants of amyloid precursor protein (APP), presenilin 1 (PS1) and presenilin 2 (PS2) genes are known, and while they generally induce early onset dementia, the incidence of early onset dementia is more or less 1% of all types of Alzheimer's dementia. The apolipoprotein E4 genotype (allele) is a genetic risk factor that increases dementia incidence approximately 10 to 35% in elderly people 65 years or older. While many researchers have recognized that Alzheimer's dementia is a complex disease caused by various non-genetic environmental factors such as aging, stress, etc., other than genetic factors, the mechanism of Alzheimer's dementia caused by non-genetic and environmental causes is not yet well known. Early onset Alzheimer's dementia caused by a genetic cause may occur in people in their 20s to 40s, which is exceptional, and most dementia cases are diagnosed at 65 years or older.

Although Alzheimer's dementia is a degenerative nervous system disease accompanied by neuropathological findings, today, it is clinically diagnosed by neuropsychological and psychological tests, and the diagnostic accuracy is 80 to 90%. In addition, there is a disadvantage in that potential dementia patients or dementia patients have a severe psychological rejection of repeated diagnosis by the above-mentioned methods. While it is possible to diagnose Alzheimer's dementia by visually detecting β-amyloid deposited in the brain by fMRI, this method is currently used only at the research level. In addition, according to studies of visually detecting the β-amyloid deposited in the brain of Alzheimer's dementia patients and normal persons using fMRI, it was reported that an actual percentage of determining Alzheimer's dementia among all the cases with imaging findings of the deposition of β-amyloid in the brain is approximately 80 to 85%, and 10% or more of normal persons also show the imaging findings of β-amyloid deposition. Taken together, it seems that the method of visually detecting β-amyloid using fMRI is not the best way to diagnose Alzheimer's dementia. Globally, the number of Alzheimer's dementia patients is increasing rapidly due to the increase in the elderly population, but the most common method of diagnosing Alzheimer's dementia still depends on neuropsychiatric psychological testing. Therefore, there is a highly urgent need for the development of a novel diagnostic method which is healthy and medical-friendly in terms of cost and technology (Korean Patent No. 10-0595494).

Meanwhile, it is known that the number of microorganisms symbiotically living in the human body is 100 trillion which is 10 times the number of human cells, and the number of genes of microorganisms exceeds 100 times the number of human genes. A microbiota is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment. Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, in the onset of Alzheimer's dementia, identification of causative factors of Alzheimer's dementia through metagenomic analysis of bacteria-derived vesicles isolated from a human-derived substance, such as blood or urine and the like, and a method of predicting or diagnosing Alzheimer's dementia have never been reported.

### [Disclosure]

### [Technical Problem]

The present inventors extracted genes from bacteria-derived extracellular vesicles present in blood as normal individual-derived and subject-derived samples and performed a metagenomic analysis in this regard in order to diagnose the causal factors and risk of Alzheimer's dementia in advance, and as a result, identified bacteria-derived extracellular vesicles which may act as a causal factor of Alzheimer's dementia, thereby completing the present invention based on this.

Therefore, an object of the present invention is to provide a method of providing information for diagnosing Alzheimer's dementia, a method of diagnosing Alzheimer's dementia, a method of predicting the risk of Alzheimer's dementia onset, and the like through the metagenomic analysis of bacteria-derived extracellular vesicles.

In addition, an object of the present invention is to provide a method of providing information for diagnosing mild cognitive impairment, a method of diagnosing mild cognitive impairment, a method of predicting the risk of mild cognitive impairment onset, and the like through the metagenomic analysis of bacteria-derived extracellular vesicles.

However, the technical goals of the present invention are not limited to the aforementioned goals, and other unmentioned technical goals will be clearly understood by those of ordinary skill in the art from the following description.

### [Technical Solution]

To achieve the above-described object of the present invention, there is provided a method of providing information for Alzheimer's dementia diagnosis, comprising the following processes:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The present invention also provides a method of diagnosing Alzheimer's dementia, comprising the following processes:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The present invention also provides a method of predicting a risk for Alzheimer's dementia, comprising the following processes:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

In one embodiment of the present invention, process (c) may comprise comparing an increase or decrease in content of extracellular vesicles derived from one or more bacteria selected from the group consisting of the phylum *Deferribacteres,* the phylum SRI, the phylum *Synergistetes,* and the phylum *Thermi.*

In another embodiment of the present invention, process (c) may comprise comparing an increase or decrease in content of extracellular vesicles derived from one or more bacteria selected from the group consisting of the class *Alphaproteobacteria,* the class *Flavobacteriia,* the class *Deferribacteres,* and the class *Deinococci.*

In another embodiment of the present invention, process (c) may comprise comparing an increase or decrease in content of extracellular vesicles derived from bacteria of the order *Rickettsiales.*

In another embodiment of the present invention, process (c) may comprise comparing an increase or decrease in content of extracellular vesicles derived from one or more bacteria selected from the group consisting of the family *Sphingomonadaceae,* the family *Deferribacteraceae,* the family *Weeksellaceae,* the family *Peptococcaceae,* the family *Rhodobacteraceae,* the family *Nocardiaceae,* the family *Neisseriaceae,* the family *Tissierellaceae,* the family *Flavobacteriaceae,* the family *Paraprevotellaceae,* the family *Oxalobacteraceae,* the family *Gemellaceae,* the family *Aerococcaceae,* the family *Leptotrichiaceae,* the family *Rhodocyclaceae,* the family *Williamsiaceae,* and the family *Deinococcaceae.*

In another embodiment of the present invention, process (c) may comprise comparing an increase or decrease in content of extracellular vesicles derived from one or more bacteria selected from the group consisting of the genus *Sphingomonas,* the genus *Mucispirillum,* the genus *Cloacibacterium,* the genus rc4-4, the genus *Collinsella,* the genus *Rothia,* the genus *Dechloromonas,* the genus *Rhodococcus,* the genus *Neisseria,* the genus *Paracoccus,* the genus *Citrobacter,* the genus *Porphyromonas,* the genus *Anaerococcus,* the genus *Prevotella,* the genus *Tepidimonas,* the genus *Leptotrichia,* the genus *Capnocytophaga,* the genus *Adlercreutzia,* the genus *Williamsia,* and the genus *Deinococcus.*

In one embodiment of the present invention, the normal individual and subject samples are blood, and
process (c) may comprise comparing an increase or decrease in content of extracellular vesicles derived from one or more bacteria selected from the group consisting of the phylum *Deferribacteres,* the phylum SRI, the phylum *Synergistetes,* and the phylum *Thermi*;
extracellular vesicles derived from one or more bacteria selected from the group consisting of the class *Alphaproteobacteria,* the class *Flavobacteriia,* the class *Deferribacteres,* and the class *Deinococci*;
extracellular vesicles derived from bacteria of the order *Rickettsiales,*
extracellular vesicles derived from one or more bacteria selected from the group consisting of the family *Sphingomonadaceae,* the family *Deferribacteraceae,* the family *Weeksellaceae,* the family *Peptococcaceae,* the family *Rhodobacteraceae,* the family *Nocardiaceae,* the family *Neisseriaceae,* the family *Tissierellaceae,* the family *Flavobacteriaceae,* the family *Paraprevotellaceae,* the family *Oxalobacteraceae,* the family *Gemellaceae,* the family *Aerococcaceae,* the family *Leptotrichiaceae,* the family *Rhodocyclaceae,* the family *Williamsiaceae,* and the family *Deinococcaceae*; or
extracellular vesicles derived from one or more bacteria selected from the group consisting of the genus *Sphingomonas,* the genus *Mucispirillum,* the genus *Cloacibacterium,* the genus rc4-4, the genus *Collinsella,* the genus *Rothia,* the genus *Dechloromonas,* the genus *Rhodococcus,* the genus *Neisseria,* the genus *Paracoccus,* the genus *Citrobacter,* the genus *Porphyromonas,* the genus *Anaerococcus,* the genus *Prevotella,* the genus *Tepidimonas,* the genus *Leptotrichia,* the genus *Capnocytophaga,* the genus *Adlercreutzia,* the genus *Williamsia,* and the genus *Deinococcus.*

In another embodiment of the present invention, in process (c), in comparison with the normal individual-derived sample, it is possible to diagnose an increase in the content of the following as Alzheimer's dementia:
extracellular vesicles derived from bacteria of the phylum *Deferribacteres,*
extracellular vesicles derived from one or more bacteria selected from the group consisting of the class *Alphaproteobacteria* and the class *Deferribacteres,*
extracellular vesicles derived from one or more bacteria selected from the group consisting of the family *Sphingomonadaceae,* the family *Deferribacteraceae,* the family *Peptococcaceae,* the family *Rhodobacteraceae,* the family *Paraprevotellaceae,* the family *Gemellaceae,* the family *Leptotrichiaceae,* and the family *Williamsiaceae,* or
extracellular vesicles derived from one or more bacteria selected from the group consisting of the genus *Sphingomonas,* the genus *Mucispirillum,* the genus rc4-4, the genus *Paracoccus,* the genus *Porphyromonas,* the genus *Prevotella,* the genus *Tepidimonas,* the genus *Leptotrichia,* the genus *Adlercreutzia,* the genus *Williamsia.*

In another embodiment of the present invention, in process (c), in comparison with the normal individual-derived sample, it is possible to diagnose a decrease in the content of the following as Alzheimer's dementia:
extracellular vesicles derived from one or more bacteria selected from the group consisting of the phylum SRI, the phylum *Synergistetes*, and the phylum *Thermi,*
extracellular vesicles derived from one or more bacteria selected from the group consisting of the class *Flavobacteriia* and the class *Deinococci,*
extracellular vesicles derived from bacteria of the order *Rickettsiales,*
extracellular vesicles derived from one or more bacteria selected from the group consisting of the family *Weeksellaceae,* the family *Nocardiaceae,* the family *Neisseriaceae,* the family *Tissierellaceae,* the family *Flavobacteriaceae,* the family *Oxalobacteraceae,* the family *Aerococcaceae,* the family *Rhodocyclaceae,* the family *Deinococcaceae,*
extracellular vesicles derived from one or more bacteria selected from the group consisting of the genus *Cloacibacterium,* the genus *Collinsella,* the genus *Rothia,* the genus *Dechloromonas,* the genus *Rhodococcus,* the genus *Neisseria,* the genus *Citrobacter,* the genus *Anaerococcus,* the genus *Capnocytophaga,* the genus *Deinococcus.*

To achieve the above-described object of the present invention, there is provided a method of providing information for mild cognitive impairment diagnosis, comprising the following processes:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The present invention also provides a method of diagnosing mild cognitive impairment, comprising the following processes:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

The present invention also provides a method of predicting a risk for mild cognitive impairment, comprising the following processes:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

In one embodiment of the present invention, process (c) may comprise comparing an increase or decrease in content of extracellular vesicles derived from one or more bacteria selected from the group consisting of the phylum *Fusobacteria,* the phylum *Cyanobacteria,* the phylum SRI, the phylum TM7, the phylum *Thermi,* the phylum *Chloroflexi,* the phylum *Armatimonadetes.*

In another embodiment of the present invention, process (c) may comprise comparing an increase or decrease in content of extracellular vesicles derived from one or more bacteria selected from the group consisting of the class *Betaproteobacteria,* the class *Fusobacteriia,* the class *Chloroplast,* the class TM7-3, the class *Deinococci,* the class *Fimbriimonadia.*

In another embodiment of the present invention, process (c) may comprise comparing an increase or decrease in content of extracellular vesicles derived from one or more bacteria selected from the group consisting of the order *Streptophyta* and the order *Rickettsiales.*

In another embodiment of the present invention, process (c) may comprise comparing an increase or decrease in content of extracellular vesicles derived from one or more bacteria selected from the group consisting of the family *Weeksellaceae,* the family *Fusobacteriaceae,* the family *Xanthomonadaceae,* the family *Rhodocyclaceae,* the family *Odoribacteraceae,* the family *Rhodobacteraceae,* the family *Nocardiaceae,* the family *Oxalobacteraceae,* the family *Microbacteriaceae,* the family *Deinococcaceae,* the family *Paenibacillaceae,* the family *Rhizobiaceae,* the family *Fimbriimonadaceae.*

In another embodiment of the present invention, process (c) may comprise comparing an increase or decrease in content of extracellular vesicles derived from one or more bacteria selected from the group consisting of the genus *Cloacibacterium,* the genus *Fusobacterium,* the genus *Lactococcus,* the genus *Stenotrophomonas,* the genus *Dechloromonas,* the genus *Odoribacter,* the genus *Rhodococcus,* the genus *Flavobacterium,* the genus *Deinococcus,* the genus *Paenibacillus,* the genus *Citrobacter,* the genus *Fimbriimonas.*

In one embodiment of the present invention, the normal individual and subject samples are blood, and
process (c) may comprise comparing an increase or decrease in content of extracellular vesicles derived from one or more bacteria selected from the group consisting of the phylum *Fusobacteria,* the phylum *Cyanobacteria,* the phylum SRI, the phylum TM7, the phylum *Thermi,* the phylum *Chloroflexi,* and the phylum *Armatimonadetes;*
extracellular vesicles derived from one or more bacteria selected from the group consisting of the class *Betaproteobacteria,* the class *Fusobacteriia,* the class *Chloroplast,* the class TM7-3, the class *Deinococci,* and the class *Fimbriimonadia*;
extracellular vesicles derived from one or more bacteria selected from the group consisting of the order *Streptophyta* and the order *Rickettsiales*;
extracellular vesicles derived from one or more bacteria selected from the group consisting of the family *Weeksellaceae,* the family *Fusobacteriaceae,* the family *Xanthomonadaceae,* the family *Rhodocyclaceae,* the family *Odoribacteraceae,* the family *Rhodobacteraceae,* the family *Nocardiaceae,* the family *Oxalobacteraceae,* the family *Microbacteriaceae,* the family *Deinococcaceae,* the family *Paenibacillaceae,* the family *Rhizobiaceae,* and the family *Fimbriimonadaceae*; or
extracellular vesicles derived from one or more bacteria selected from the group consisting of the genus *Cloacibacterium,* the genus *Fusobacterium,* the genus *Lactococcus,* the genus *Stenotrophomonas,* the genus *Dechloromonas,* the genus *Odoribacter,* the genus *Rhodococcus,* the genus *Flavobacterium,* the genus *Deinococcus,* the genus *Paenibacillus,* the genus *Citrobacter,* and the genus *Fimbriimonas.*

In another embodiment of the present invention, in process (c), in comparison with the normal individual-derived sample, it is possible to diagnose an increase in the content of the following as mild cognitive impairment:
extracellular vesicles derived from bacteria of the phylum *Fusobacteria,*
extracellular vesicles derived from bacteria of the class *Fusobacteriia,*
extracellular vesicles derived from one or more bacteria selected from the group consisting of the family *Fusobacteriaceae,* the family *Odoribacteraceae,* the family *Rhodobacteraceae,* the family *Microbacteriaceae,* the family *Paenibacillaceae,* and the family *Rhizobiaceae,* or
extracellular vesicles derived from one or more bacteria selected from the group consisting of the genus *Fusobacterium,* the genus *Lactococcus,* the genus *Odoribacter,* the genus *Flavobacterium,* and the genus *Paenibacillus.*

In another embodiment of the present invention, in process (c), in comparison with the normal individual-derived sample, it is possible to diagnose a decrease in the content of the following as mild cognitive impairment:
extracellular vesicles derived from one or more bacteria selected from the group consisting of the phylum *Cyanobacteria,* the phylum SRI, the phylum TM7, the phylum *Thermi,* the phylum *Chloroflexi,* and the phylum *Armatimonadetes,*
extracellular vesicles derived from one or more bacteria selected from the group consisting of the class *Betaproteobacteria,* the class *Chloroplast,* the class TM7-3, the class *Deinococci,* and the class *Fimbriimonadia,*
extracellular vesicles derived from one or more bacteria selected from the group consisting of the order *Streptophyta* and the order *Rickettsiales,*
extracellular vesicles derived from one or more bacteria selected from the group consisting of the family *Weeksellaceae,* the family *Xanthomonadaceae,* the family *Rhodocyclaceae,* the family *Nocardiaceae,* the family *Oxalobacteraceae,* the family *Deinococcaceae,* and the family *Fimbriimonadaceae,* or
extracellular vesicles derived from one or more bacteria selected from the group consisting of the genus *Cloacibacterium,* the genus *Stenotrophomonas,* the genus *Dechloromonas,* the genus *Rhodococcus,* the genus *Deinococcus,* the genus *Citrobacter,* and the genus *Fimbriimonas.*

To achieve the above-described object of the present invention, there is provided a method of providing information for Alzheimer's dementia diagnosis, comprising the following processes:
(a) extracting DNAs from extracellular vesicles isolated from mild cognitive impairment patient and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a mild cognitive impairment patient-derived sample through sequencing of a product of the PCR.

The present invention also provides a method of diagnosing Alzheimer's dementia, comprising the following processes:
(a) extracting DNAs from extracellular vesicles isolated from mild cognitive impairment patient and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a mild cognitive impairment patient-derived sample through sequencing of a product of the PCR.

The present invention also provides a method of predicting a risk for Alzheimer's dementia, comprising the following processes:
(a) extracting DNAs from extracellular vesicles isolated from mild cognitive impairment patient and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a mild cognitive impairment patient-derived sample through sequencing of a product of the PCR.

In one embodiment of the present invention, process (c) may comprise comparing an increase or decrease in content of extracellular vesicles derived from one or more bacteria selected from the group consisting of the phylum *Fusobacteria,* the phylum *Deferribacteres,* and the phylum *Armatimonadetes.*

In another embodiment of the present invention, process (c) may comprise comparing an increase or decrease in content of extracellular vesicles derived from one or more bacteria selected from the group consisting of the class *Fusobacteriia,* the class *Deferribacteres,* and the class *Alphaproteobacteria.*

In another embodiment of the present invention, process (c) may comprise comparing an increase or decrease in content of extracellular vesicles derived from bacteria of the order *Methanobacteriales.*

In another embodiment of the present invention, process (c) may comprise comparing an increase or decrease in content of extracellular vesicles derived from one or more bacteria selected from the group consisting of the family *Microbacteriaceae,* the family *Fusobacteriaceae,* the family *Aerococcaceae,* the family *Bifidobacteriaceae,* the family *Deferribacteraceae,* the family *Sphingomonadaceae,* the family *Flavobacteriaceae,* the family *Rhizobiaceae,* the family *Leptotrichiaceae,* and the family *Micrococcaceae*

In another embodiment of the present invention, process (c) may comprise comparing an increase or decrease in content of extracellular vesicles derived from one or more bacteria selected from the group consisting of the genus *Fusobacterium,* the genus *Collinsella,* the genus *Sphingomonas,* the genus *Bifidobacterium,* the genus *Mucispirillum,* the genus *Paracoccus,* the genus *Flavobacterium,* the genus *Blautia,* the genus *Tepidimonas,* the genus *Odoribacter,* the genus *Veillonella,* the genus *Porphyromonas,* and the genus *Leptotrichia.*

In one embodiment of the present invention, the mild cognitive impairment patient and subject samples are blood, and
process (c) may comprise comparing an increase or decrease in content of extracellular vesicles derived from one or more bacteria selected from the group consisting of the phylum *Fusobacteria,* the phylum *Deferribacteres,* and the phylum *Armatimonadetes;*
extracellular vesicles derived from one or more bacteria selected from the group consisting of the class *Fusobacteriia,* the class *Deferribacteres,* and the class *Alphaproteobacteria*;
extracellular vesicles derived from bacteria of the order *Methanobacteriales*;
extracellular vesicles derived from one or more bacteria selected from the group consisting of the family *Microbacteriaceae,* the family *Fusobacteriaceae,* the family *Aerococcaceae,* the family *Bifidobacteriaceae,* the family *Deferribacteraceae,* the family *Sphingomonadaceae,* the family *Flavobacteriaceae,* the family *Rhizobiaceae,* the family *Leptotrichiaceae,* and the family *Micrococcaceae*; or
extracellular vesicles derived from one or more bacteria selected from the group consisting of the genus *Fusobacterium,* the genus *Collinsella,* the genus *Sphingomonas,* the genus *Bifidobacterium,* the genus *Mucispirillum,* the genus *Paracoccus,* the genus *Flavobacterium,* the genus *Blautia,* the genus *Tepidimonas,* the genus *Odoribacter,* the genus *Veillonella,* the genus *Porphyromonas,* and the genus *Leptotrichia.*

In another embodiment of the present invention, in process (c), in comparison with the mild cognitive impairment patient-derived sample, it is possible to diagnose an increase in the content of the following as Alzheimer's dementia:
extracellular vesicles derived from one or more bacteria selected from the group consisting of the phylum *Deferribacteres* and the phylum *Armatimonadetes,*
extracellular vesicles derived from one or more bacteria selected from the group consisting of the class *Deferribacteres* and the class *Alphaproteobacteria,*
extracellular vesicles derived from one or more bacteria selected from the group consisting of the family *Deferribacteraceae,* the family *Sphingomonadaceae,* and the family *Leptotrichiaceae,* or
extracellular vesicles derived from one or more bacteria selected from the group consisting of the genus *Sphingomonas,* the genus *Mucispirillum,* the genus *Paracoccus,* the genus *Tepidimonas,* the genus *Porphyromonas,* and the genus *Leptotrichia.*

In another embodiment of the present invention, in process (c), in comparison with the mild cognitive impairment patient-derived sample, it is possible to diagnose an decrease in the content of the following as Alzheimer's dementia:
extracellular vesicles derived from bacteria of the phylum *Fusobacteria,*
extracellular vesicles derived from bacteria of the class *Fusobacteriia,*
extracellular vesicles derived from bacteria of the order *Methanobacteriales,*
extracellular vesicles derived from one or more bacteria selected from the group consisting of the family *Microbacteriaceae,* the family *Fusobacteriaceae,* the family *Aerococcaceae,* the family *Bifidobacteriaceae,* the family *Flavobacteriaceae,* the family *Rhizobiaceae,* and the family *Micrococcaceae,* or
extracellular vesicles derived from one or more bacteria selected from the group consisting of the genus *Fusobacterium,* the genus *Collinsella,* the genus *Bifidobacterium,* the genus *Flavobacterium,* the genus *Blautia,* the genus *Odoribacter,* and the genus *Veillonella.*

In another embodiment of the present invention, the blood may be whole blood, serum, plasma, or blood mononuclear cells.

### [Advantageous Effects]

Extracellular vesicles secreted from bacteria present in the environment can be absorbed into the body and have a direct influence on the onset of Alzheimer's dementia, and since it is difficult to enable early diagnosis of Alzheimer's dementia before symptoms are shown, it is difficult to effectively treat Alzheimer's dementia. Therefore, Alzheimer's dementia risk groups can be diagnosed and predicted early by previously diagnosing the causative factor and the risk of the onset of Alzheimer's dementia through the metagenomic analysis of bacterial extracellular vesicles using a human-derived sample according to the present invention, and the time of onset can be delayed or the onset of the disease can be prevented with proper care. In addition, since Alzheimer's dementia can be diagnosed early after onset, the incidence of Alzheimer's dementia can be reduced and a therapeutic effect can increase, and the progress of the disease can be improved or the recurrence of the disease can be prevented by identifying causative factors through a metagenomic analysis on patients diagnosed with Alzheimer's dementia to avoid exposure to the relevant factors. Moreover, as a risk group of mild cognitive impairment can also be diagnosed early by metagenomic analysis, the time of onset can be delayed or the onset of the disease can be prevented by proper cure, and after the onset of the disease, it can be diagnosed early, and thus the incidence of the mild cognitive impairment can be reduced, and the therapeutic effect can increase.

### [Description of Drawings]

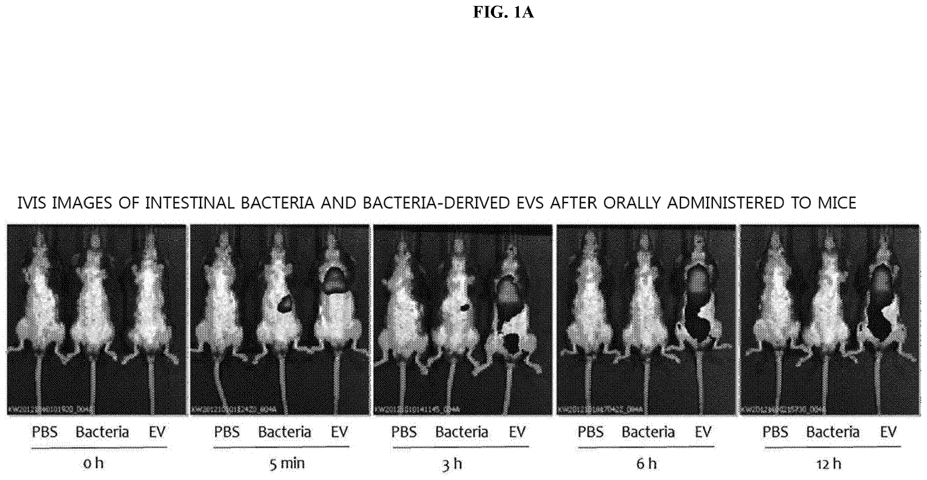

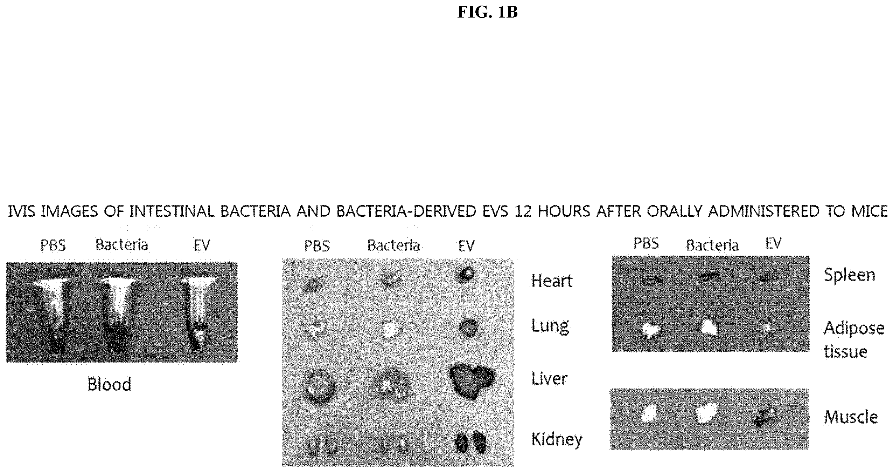

FIG. 1A illustrates images showing the distribution pattern of bacteria and extracellular vesicles over time after intestinal bacteria and bacteria-derived extracellular vesicles (EVs) were orally administered to mice, and FIG. 1B illustrates images showing the distribution pattern of bacteria and EVs after being orally administered to mice and, at 12 hours, blood and various organs were extracted.

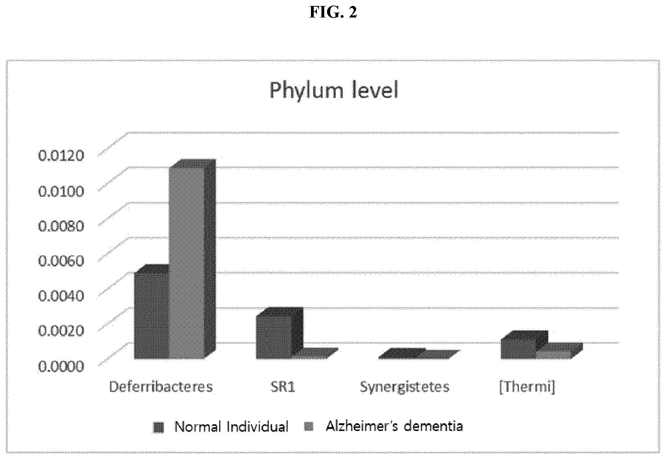

FIG. 2 is a result showing the distribution of bacteria-derived extracellular vesicles (EVs), which is significant in diagnostic performance at the phylum level by isolating bacteria-derived vesicles from blood of a patient with Alzheimer's dementia and a normal individual, and then performing a metagenomic analysis.

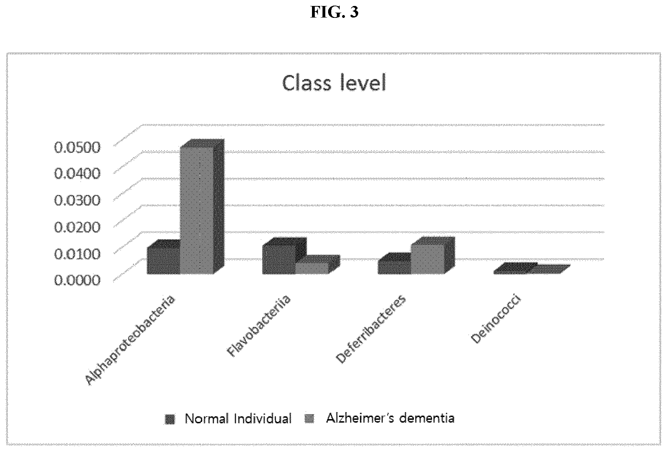

FIG. 3 is a result showing the distribution of bacteria-derived extracellular vesicles (EVs), which is significant in diagnostic performance at the class level by isolating bacteria-derived vesicles from blood of a patient with Alzheimer's dementia and a normal individual, and then performing a metagenomic analysis.

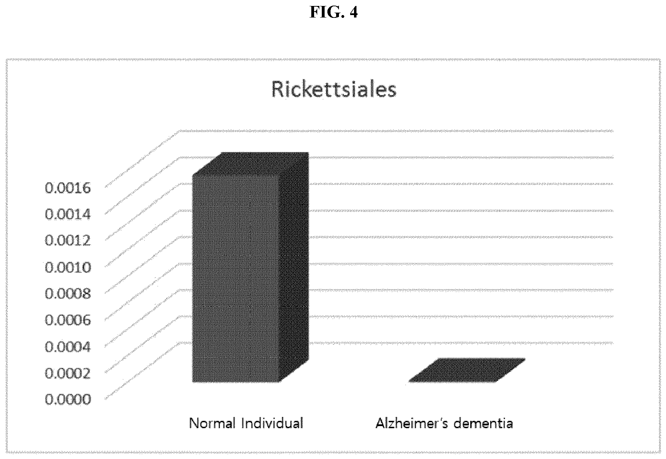

FIG. 4 is a result showing the distribution of bacteria-derived extracellular vesicles (EVs), which is significant in diagnostic performance at the order level by isolating bacteria-derived vesicles from blood of a patient with Alzheimer's dementia and a normal individual, and then performing a metagenomic analysis.

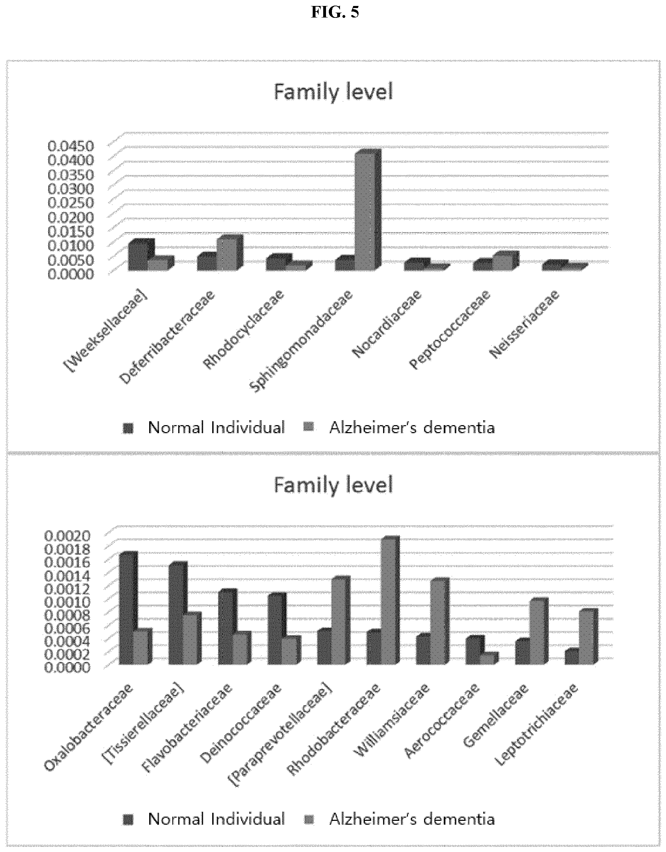

FIG. 5 is a result showing the distribution of bacteria-derived extracellular vesicles (EVs), which is significant in diagnostic performance at the family level by isolating bacteria-derived vesicles from blood of a patient with Alzheimer's dementia and a normal individual, and then performing a metagenomic analysis.

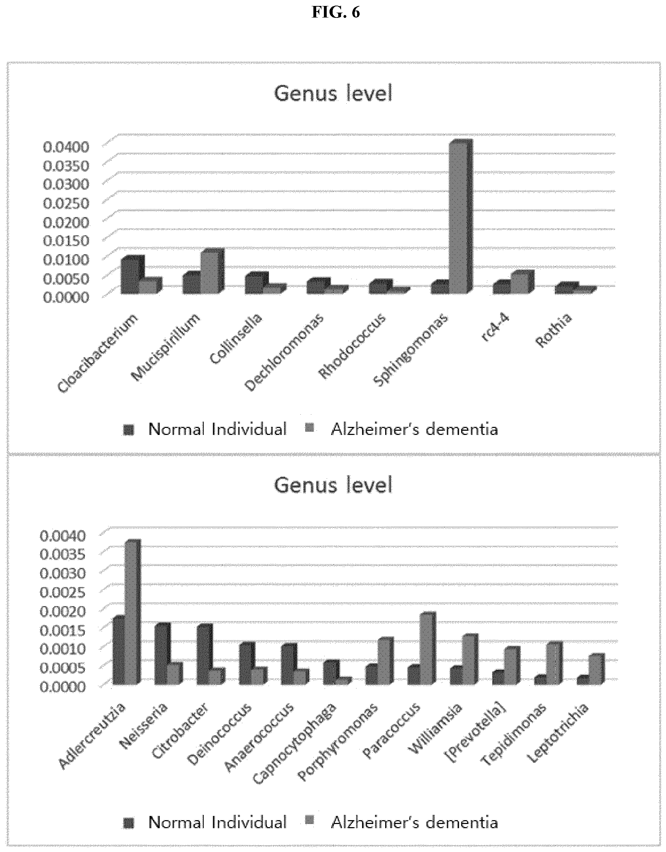

FIG. 6 is a result showing the distribution of bacteria-derived extracellular vesicles (EVs), which is significant in diagnostic performance at the genus level by isolating bacteria-derived vesicles from blood of a patient with Alzheimer's dementia and a normal individual, and then performing a metagenomic analysis.

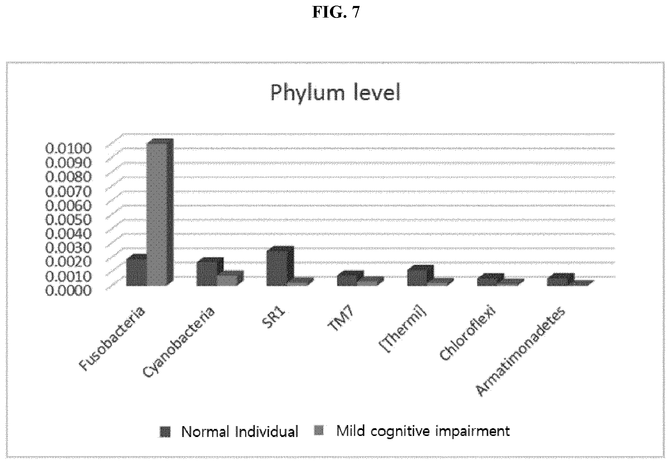

FIG. 7 is a result showing the distribution of bacteria-derived extracellular vesicles (EVs), which is significant in diagnostic performance at the phylum level by isolating bacteria-derived vesicles from blood of a patient with mild cognitive impairment and a normal individual, and then performing a metagenomic analysis.

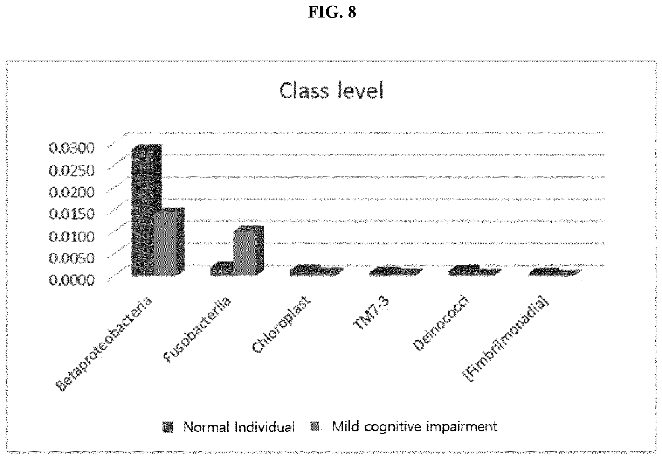

FIG. 8 is a result showing the distribution of bacteria-derived extracellular vesicles (EVs), which is significant in diagnostic performance at the class level by isolating bacteria-derived vesicles from blood of a patient with mild cognitive impairment and a normal individual, and then performing a metagenomic analysis.

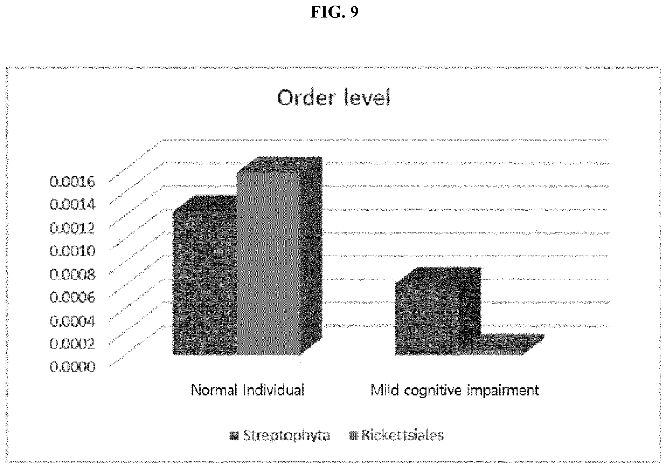

FIG. 9 is a result showing the distribution of bacteria-derived extracellular vesicles (EVs), which is significant in diagnostic performance at the order level by isolating bacteria-derived vesicles from blood of a patient with mild cognitive impairment and a normal individual, and then performing a metagenomic analysis.

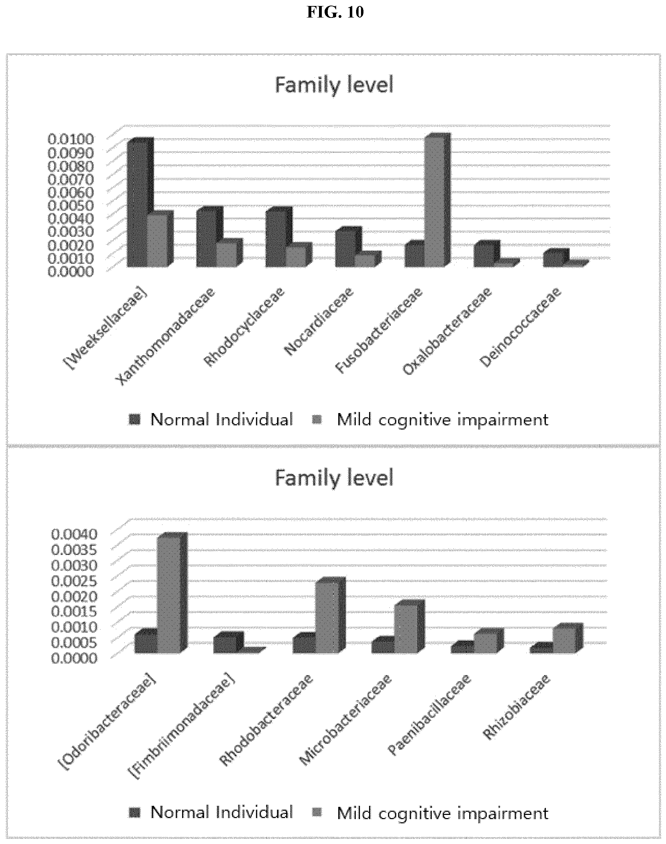

FIG. 10 is a result showing the distribution of bacteria-derived extracellular vesicles (EVs), which is significant in diagnostic performance at the family level by isolating bacteria-derived vesicles from blood of a patient with mild cognitive impairment and a normal individual, and then performing a metagenomic analysis.

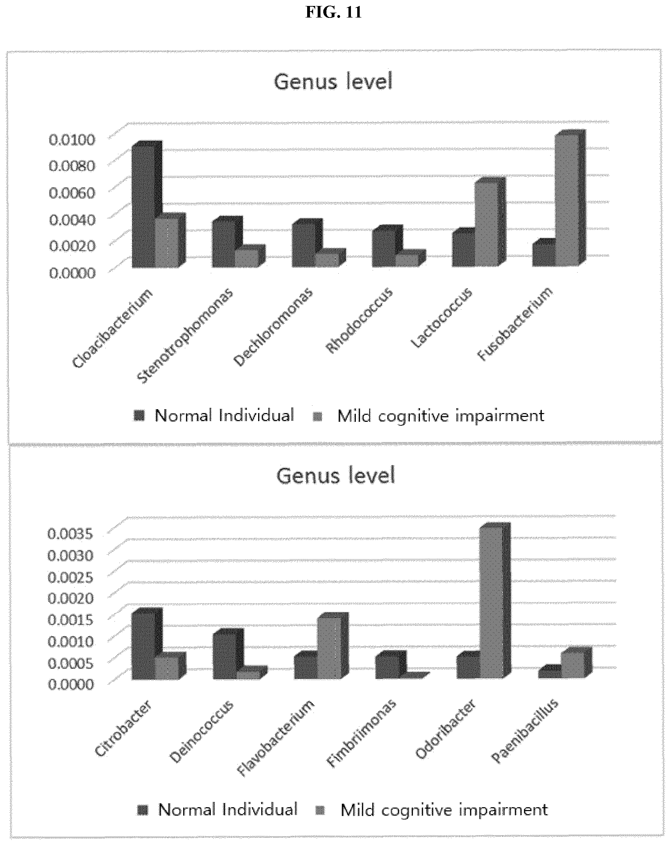

FIG. 11 is a result showing the distribution of bacteria-derived extracellular vesicles (EVs), which is significant in diagnostic performance at the genus level by isolating bacteria-derived vesicles from blood of a patient with mild cognitive impairment and a normal individual, and then performing a metagenomic analysis.

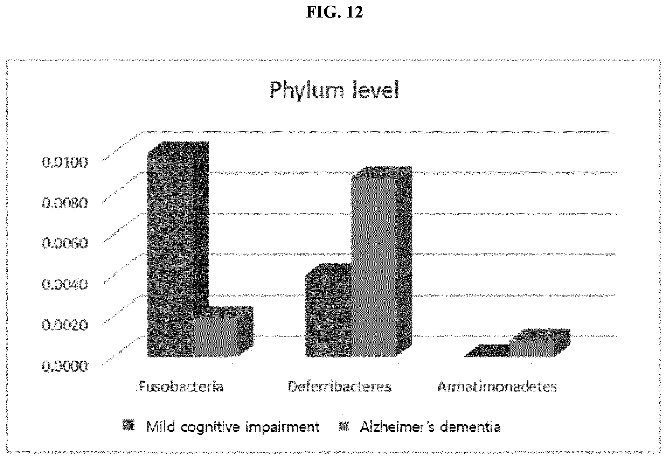

FIG. 12 is a result showing the distribution of bacteria-derived extracellular vesicles (EVs), which is significant in diagnostic performance at the phylum level by isolating bacteria-derived vesicles from blood of a patient with mild cognitive impairment and a patient with Alzheimer's dementia, and then performing a metagenomic analysis.

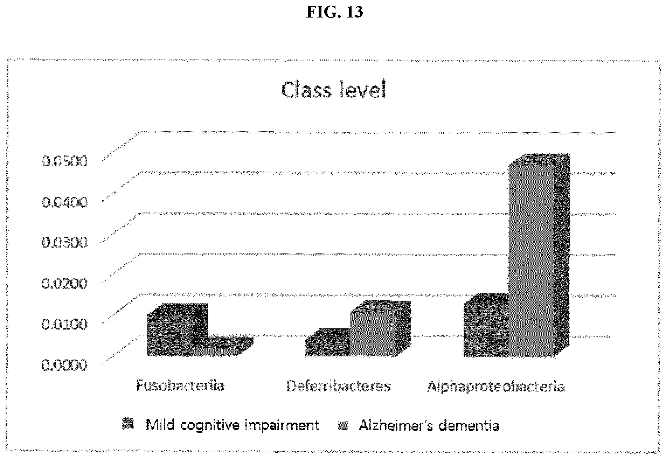

FIG. 13 is a result showing the distribution of bacteria-derived extracellular vesicles (EVs), which is significant in diagnostic performance at the class level by isolating bacteria-derived vesicles from blood of a patient with mild cognitive impairment and a patient with Alzheimer's dementia, and then performing a metagenomic analysis.

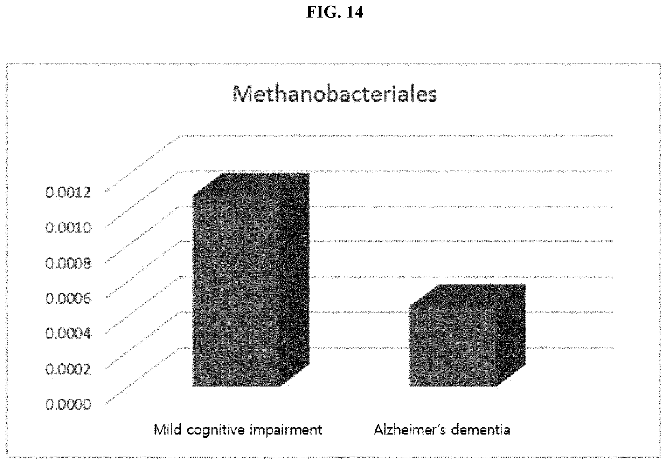

FIG. 14 is a result showing the distribution of bacteria-derived extracellular vesicles (EVs), which is significant in diagnostic performance at the order level by isolating bacteria-derived vesicles from blood of a patient with mild cognitive impairment and a patient with Alzheimer's dementia, and then performing a metagenomic analysis.

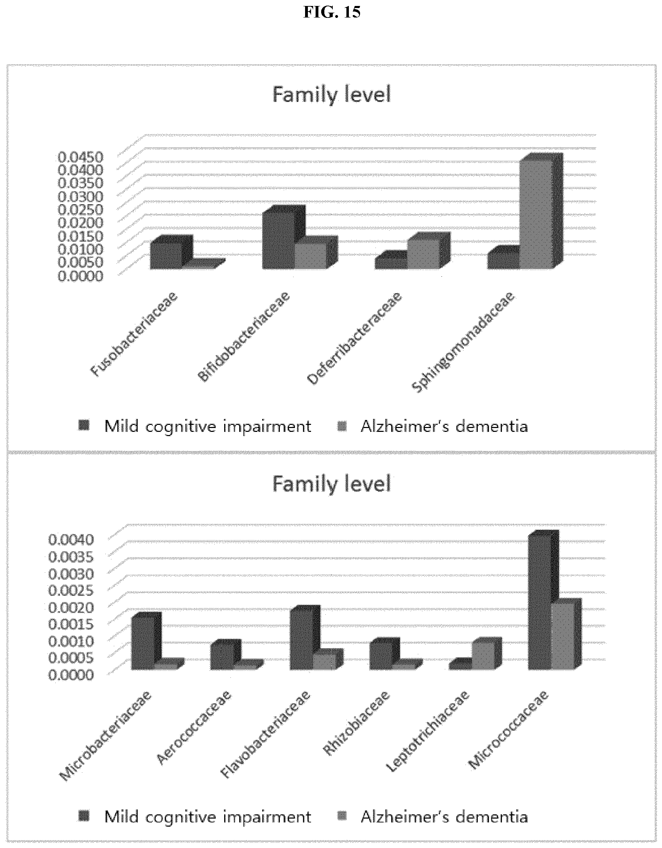

FIG. 15 is a result showing the distribution of bacteria-derived extracellular vesicles (EVs), which is significant in diagnostic performance at the family level by isolating bacteria-derived vesicles from blood of a patient with mild cognitive impairment and a patient with Alzheimer's dementia, and then performing a metagenomic analysis.

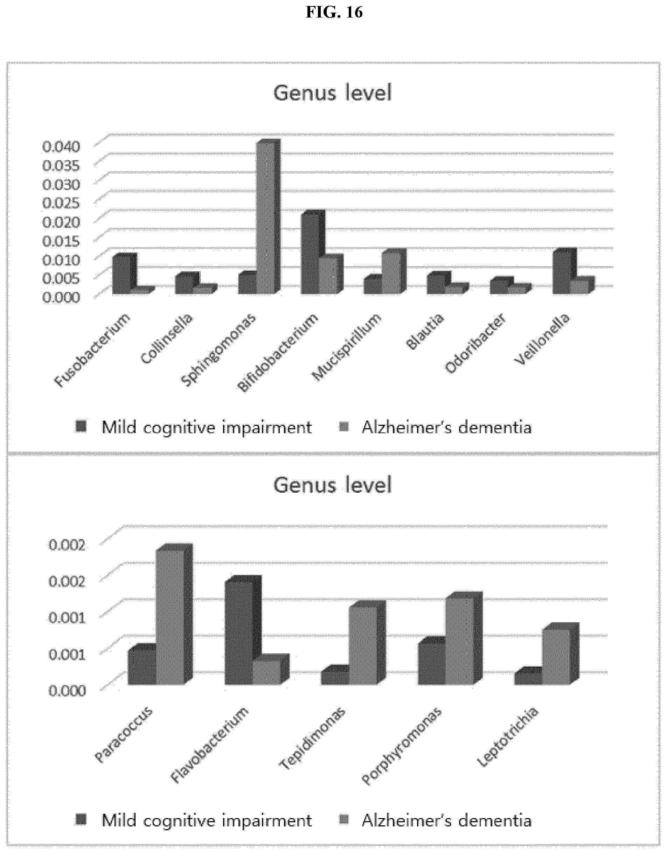

FIG. 16 is a result showing the distribution of bacteria-derived extracellular vesicles (EVs), which is significant in diagnostic performance at the genus level by isolating bacteria-derived vesicles from blood of a patient with mild cognitive impairment and a patient with Alzheimer's dementia, and then performing a metagenomic analysis.

### [Best Mode]

The present invention relates to a method of diagnosing Alzheimer's dementia and mild cognitive impairment through bacterial metagenomic analysis. The inventors of the present invention extracted genes from bacteria-derived extracellular vesicles using a normal individual and a subject-derived sample, performed metagenomic analysis thereon, and identified bacteria-derived extracellular vesicles capable of acting as a causative factor of Alzheimer's dementia and mild cognitive impairment.

Therefore, the present invention provides a method of providing information for diagnosing Alzheimer's dementia, the method comprising:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

In addition, the present invention provides a method of providing information for diagnosing mild cognitive impairment, the method comprising:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

In addition, the present invention provides a method of providing information for diagnosing Alzheimer's dementia, the method comprising:
(a) extracting DNAs from extracellular vesicles isolated from mild cognitive impairment patient and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a mild cognitive impairment patient-derived sample through sequencing of a product of the PCR.

The term "Alzheimer's dementia diagnosis" as used herein refers to determining whether a patient has a risk for Alzheimer's dementia, whether the risk for Alzheimer's dementia is relatively high, or whether Alzheimer's dementia has already occurred. The method of the present invention may be used to delay the onset of Alzheimer's dementia through special and appropriate care for a specific patient, which is a patient having a high risk for Alzheimer's dementia or prevent the onset of Alzheimer's dementia. In addition, the method may be clinically used to determine treatment by selecting the most appropriate treatment method through early diagnosis of Alzheimer's dementia.

The term "mild cognitive impairment" used herein refers to a decline in cognitive ability due to a normal aging phenomenon, and is defined as a state in which the cognitive ability has declined, compared to the same age group. The difference between the mild cognitive impairment and dementia is that everyday life is possible, and since it is known that 10% of elderly patients with mild cognitive impairment develop dementia, the mild cognitive impairment is known as a risk factor of dementia.

The term "mild cognitive impairment diagnosis" as used herein refers to determining whether a patient has a risk for mild cognitive impairment, whether the risk for mild cognitive impairment is relatively high, or whether mild cognitive impairment has already occurred. The method of the present invention may be used to delay the onset of mild cognitive impairment through special and appropriate care for a specific patient, which is a patient having a high risk for mild cognitive impairment or prevent the onset of mild cognitive impairment. In addition, the method may be clinically used to determine treatment by selecting the most appropriate treatment method through early diagnosis of mild cognitive impairment.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, blood.

In the present invention, the normal individual and subject sample may be blood or urine, and the blood may be preferably whole blood, serum, plasma, or blood mononuclear cells, but the present invention is not limited thereto.

In an embodiment of the present invention, metagenomic analysis is performed on the bacteria-derived extracellular vesicles, and bacteria-derived extracellular vesicles capable of acting as a cause of the onset of Alzheimer's dementia were actually identified by analysis at phylum, class, order, family, and genus levels.

More particularly, in one embodiment of the present invention, as a result of performing bacterial metagenomic analysis on extracellular vesicles present in subject-derived blood samples at a phylum level, the content of extracellular vesicles derived from bacteria belonging to the phylum *Deferribacteres,* the phylum SRI, the phylum *Synergistetes,* and the phylum *Thermiwas* significantly different between Alzheimer's dementia patients and normal individuals (see Example 4).

More particularly, in one embodiment of the present invention, as a result of performing bacterial metagenomic analysis on extracellular vesicles present in subject-derived blood samples at a class level, the content of extracellular vesicles derived from bacteria belonging to the class *Alphaproteobacteria,* the class *Flavobacteriia,* the class *Deferribacteres,* and the class *Deinococci* significantly different between Alzheimer's dementia patients and normal individuals (see Example 4).

More particularly, in one embodiment of the present invention, as a result of performing bacterial metagenomic analysis on extracellular vesicles present in subject-derived blood samples at an order level, the content of extracellular vesicles derived from bacteria belonging to the order *Rickettsiales* significantly different between Alzheimer's dementia patients and normal individuals (see Example 4).

More particularly, in one embodiment of the present invention, as a result of performing bacterial metagenomic analysis on extracellular vesicles present in subject-derived blood samples at a family level, the content of extracellular vesicles derived from bacteria belonging to the family *Sphingomonadaceae,* the family *Deferribacteraceae,* the family *Weeksellaceae,* the family *Peptococcaceae,* the family *Rhodobacteraceae,* the family *Nocardiaceae,* the family *Neisseriaceae,* the family *Tissierellaceae,* the family *Flavobacteriaceae,* the family *Paraprevotellaceae,* the family *Oxalobacteraceae,* the family *Gemellaceae,* the family *Aerococcaceae,* the family *Leptotrichiaceae,* the family *Rhodocyclaceae,* the family *Williamsiaceae,* and the family *Deinococcaceae* significantly different between Alzheimer's dementia patients and normal individuals (see Example 4).

More particularly, in one embodiment of the present invention, as a result of performing bacterial metagenomic analysis on extracellular vesicles present in subject-derived blood samples at a genus level, the content of extracellular vesicles derived from bacteria belonging to the genus *Sphingomonas,* the genus *Mucispirillum,* the genus *Cloacibacterium,* the genus rc4-4, the genus *Collinsella,* the genus *Rothia,* the genus *Dechloromonas,* the genus *Rhodococcus,* the genus *Neisseria,* the genus *Paracoccus,* the genus *Citrobacter,* the genus *Porphyromonas,* the genus *Anaerococcus,* the genus *Prevotella,* the genus *Tepidimonas,* the genus *Leptotrichia,* the genus *Capnocytophaga,* the genus *Adlercreutzia,* the genus *Williamsia,* and the genus *Deinococcus* significantly different between Alzheimer's dementia patients and normal individuals (see Example 4).

In another embodiment of the present invention, metagenomic analysis is performed on the bacteria-derived extracellular vesicles, and bacteria-derived extracellular vesicles capable of acting as a cause of the onset of mild cognitive impairment were actually identified by analysis at phylum, class, order, family, and genus levels.

More particularly, in one embodiment of the present invention, as a result of performing bacterial metagenomic analysis on extracellular vesicles present in subject-derived blood samples at a phylum level, the content of extracellular vesicles derived from bacteria belonging to the phylum *Fusobacteria,* the phylum *Cyanobacteria,* the phylum SRI, the phylum TM7, the phylum *Thermi,* the phylum *Chloroflexi,* and the phylum *Armatimonadetes* significantly different between mild cognitive impairment patients and normal individuals (see Example 5).

More particularly, in one embodiment of the present invention, as a result of performing bacterial metagenomic analysis on extracellular vesicles present in subject-derived blood samples at a class level, the content of extracellular vesicles derived from bacteria belonging to the class *Betaproteobacteria,* the class *Fusobacteriia,* the class *Chloroplast,* the class TM7-3, the class *Deinococci,* and the class *Fimbriimonadia* significantly different between mild cognitive impairment patients and normal individuals (see Example 5).

More particularly, in one embodiment of the present invention, as a result of performing bacterial metagenomic analysis on extracellular vesicles present in subject-derived blood samples at an order level, the content of extracellular vesicles derived from bacteria belonging to the order *Streptophyta* and the order *Rickettsiales* significantly different between mild cognitive impairment patients and normal individuals (see Example 5).

More particularly, in one embodiment of the present invention, as a result of performing bacterial metagenomic analysis on extracellular vesicles present in subject-derived blood samples at a family level, the content of extracellular vesicles derived from bacteria belonging to the family *Weeksellaceae,* the family *Fusobacteriaceae,* the family *Xanthomonadaceae,* the family *Rhodocyclaceae,* the family *Odoribacteraceae,* the family *Rhodobacteraceae,* the family *Nocardiaceae,* the family *Oxalobacteraceae,* the family *Microbacteriaceae,* the family *Deinococcaceae,* the family *Paenibacillaceae,* the family *Rhizobiaceae,* and the family *Fimbriimonadaceae* significantly different between mild cognitive impairment patients and normal individuals (see Example 5).

More particularly, in one embodiment of the present invention, as a result of performing bacterial metagenomic analysis on extracellular vesicles present in subject-derived blood samples at a genus level, the content of extracellular vesicles derived from bacteria belonging to the genus *Cloacibacterium,* the genus *Fusobacterium,* the genus *Lactococcus,* the genus *Stenotrophomonas,* the genus *Dechloromonas,* the genus *Odoribacter,* the genus *Rhodococcus,* the genus *Flavobacterium,* the genus *Deinococcus,* the genus *Paenibacillus,* the genus *Citrobacter,* and the genus *Fimbriimonas* significantly different between mild cognitive impairment patients and normal individuals (see Example 5).

In an embodiment of the present invention, metagenomic analysis is performed on the bacteria-derived extracellular vesicles, and bacteria-derived extracellular vesicles capable of acting as a cause of the onset of Alzheimer's dementia were actually identified by analysis at phylum, class, order, family, and genus levels.

More particularly, in one embodiment of the present invention, as a result of performing bacterial metagenomic analysis on extracellular vesicles present in subject-derived blood samples at a phylum level, the content of extracellular vesicles derived from bacteria belonging to the phylum *Fusobacteria,* the phylum *Deferribacteres,* and the phylum *Armatimonadetes* significantly different between Alzheimer's dementia patients and mild cognitive impairment patients (see Example 6).

More particularly, in one embodiment of the present invention, as a result of performing bacterial metagenomic analysis on extracellular vesicles present in subject-derived blood samples at a class level, the content of extracellular vesicles derived from bacteria belonging to the class *Fusobacteriia,* the class *Deferribacteres,* and the class *Alphaproteobacteria* significantly different between Alzheimer's dementia patients and mild cognitive impairment patients (see Example 6).

More particularly, in one embodiment of the present invention, as a result of performing bacterial metagenomic analysis on extracellular vesicles present in subject-derived blood samples at an order level, the content of extracellular vesicles derived from bacteria belonging to the order *Methanobacteriales* significantly different between Alzheimer's dementia patients and mild cognitive impairment patients (see Example 6).

More particularly, in one embodiment of the present invention, as a result of performing bacterial metagenomic analysis on extracellular vesicles present in subject-derived blood samples at a family level, the content of extracellular vesicles derived from bacteria belonging to the family *Microbacteriaceae,* the family *Fusobacteriaceae,* the family *Aerococcaceae,* the family *Bifidobacteriaceae,* the family *Deferribacteraceae,* the family *Sphingomonadaceae,* the family *Flavobacteriaceae,* the family *Rhizobiaceae,* the family *Leptotrichiaceae,* and the family *Micrococcaceae* significantly different between Alzheimer's dementia patients and mild cognitive impairment patients (see Example 6).

More particularly, in one embodiment of the present invention, as a result of performing bacterial metagenomic analysis on extracellular vesicles present in subject-derived blood samples at a genus level, the content of extracellular vesicles derived from bacteria belonging to the genus *Fusobacterium,* the genus *Collinsella,* the genus *Sphingomonas,* the genus *Bifidobacterium,* the genus *Mucispirillum,* the genus *Paracoccus,* the genus *Flavobacterium,* the genus *Blautia,* the genus *Tepidimonas,* the genus *Odoribacter,* the genus *Veillonella,* the genus *Porphyromonas,* and the genus *Leptotrichia* significantly different between Alzheimer's dementia patients and mild cognitive impairment patients (see Example 6).

Through the results of the examples, it was confirmed that distribution variables of the identified bacteria-derived extracellular vesicles could be usefully used for the prediction of the onset of Alzheimer's dementia or mild cognitive impairment.

### [Mode of the Invention]

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Blood

To isolate extracellular vesicles and extract DNA, from blood, first, blood was added to a 10 ml tube and centrifuged at 3,500 x g and 4 °C for 10 min to precipitate a suspension, and only a supernatant was collected, which was then placed in a new 10 ml tube. The collected supernatant was filtered using a 0.22 µm filter to remove bacteria and impurities, and then placed in centrifugal filters (50 kD) and centrifuged at 1500 x g and 4 °C for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 °C for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the blood according to the above-described method was boiled at 100 °C to allow the internal DNA to come out of the lipid and then cooled on ice. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGA G ACAGCCTACGGGNG G CWGCAG-3' | 1 |
| | 16S_V4_R | 5'- GTCTCGTGGGCTCG GAGATGTGTATAAG A GACAGGACTACHVG G GTATCTAATCC-3' | 2 |

### Example 3. Metagenomic Analysis Using DNA Extracted from Blood

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Alzheimer's dementia Diagnostic Model Based on Metagenomic Analysis of Bacteria-Derived EVs Isolated from Blood of Normal Individual and Alzheimer's dementia patient

EVs were isolated from blood samples of 67 Alzheimer's dementia patients and 70 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

As a result of analyzing bacteria-derived EVs in blood at a phylum level, a diagnostic model developed using bacteria belonging to the phylum *Deferribacteres,* the phylum SRI, the phylum *Synergistetes,* and the phylum *Thermiwas* as a biomarker exhibited significant diagnostic performance for Alzheimer's dementia (see Table 2 and FIG. 2).

**[Table 2]**

| | Normal Individual | | Alzheimer's dementia | | t-test | | Training Set | | | Test Set | | |
|---|---|---|---|---|---|---|---|---|---|---|---|---|
| Phylum | Mean | SD | Mean | SD | p-value | Ratio | AUC | sensitivi ty | specifici ty | AUC | sensitiv ity | speci ficity |
| Deferrib acteres | 0.0049 | 0.0028 | 0.0109 | 0.0088 | 0.0001 | 2.23 | 1.00 | 1.00 | 1.00 | 1.00 | 1.00 | 1.00 |
| SR1 | 0.0024 | 0.0054 | 0.0001 | 0.0004 | 0.0018 | 0.05 | 1.00 | 1.00 | 1.00 | 1.00 | 1.00 | 1.00 |
| Synergis tetes | 0.0001 | 0.0003 | 0.0000 | 0.0000 | 0.0282 | 0.00 | 1.00 | 1.00 | 1.00 | 1.00 | 1.00 | 1.00 |
| [Thermi] | 0.0011 | 0.0019 | 0.0004 | 0.0008 | 0.0127 | 0.38 | 1.00 | 1.00 | 1.00 | 0.99 | 1.00 | 0.90 |

As a result of analyzing bacteria-derived EVs in blood at a class level, a diagnostic model developed using bacteria belonging to the class *Alphaproteobacteria,* the class *Flavobacteriia,* the class *Deferribacteres,* and the class *Deinococci* as a biomarker exhibited significant diagnostic performance for Alzheimer's dementia (see Table 3 and FIG. 3).

**[Table 3]**

| | Normal Individual | | Alzheimer's dementia | | t-test | | Training Set | | | Test Set | | |
|---|---|---|---|---|---|---|---|---|---|---|---|---|
| Class | Mean | SD | Mean | SD | p-value | Ratio | AUC | sensit ivity | specif icity | AU C | sensit ivity | specif icity |
| Alphaproteo bacteria | 0.0098 | 0.0066 | 0.0470 | 0.0639 | 0.0003 | 4.78 | 1.00 | 1.00 | 1.00 | 1.00 | 1.00 | 0.90 |
| Flavobacteri ia | 0.0106 | 0.0106 | 0.0040 | 0.0030 | 0.0000 | 0.38 | 1.00 | 1.00 | 1.00 | 1.00 | 1.00 | 1.00 |
| Deferribacte res | 0.0049 | 0.0028 | 0.0109 | 0.0088 | 0.0001 | 2.23 | 1.00 | 1.00 | 1.00 | 1.00 | 1.00 | 1.00 |
| Deinococci | 0.0011 | 0.0019 | 0.0004 | 0.0008 | 0.0127 | 0.38 | 1.00 | 1.00 | 1.00 | 0.99 | 1.00 | 0.90 |

As a result of analyzing bacteria-derived EVs in blood at an order level, a diagnostic model developed using bacteria belonging to the order *Rickettsiales* as a biomarker exhibited significant diagnostic performance for Alzheimer's dementia (see Table 4 and FIG. 4).

**[Table 4]**

| | Normal Individual | | Alzheimer's dementia | | t-test | | Training Set | | | Test Set | | |
|---|---|---|---|---|---|---|---|---|---|---|---|---|
| Order | Mean | SD | Mean | SD | p-value | Ratio | AUC | sensiti vity | specifi city | AUC | sensiti vity | spec ificit y |
| Ricketts iales | 0.0016 | 0.0037 | 0.0000 | 0.0001 | 0.0023 | 0.01 | 1.00 | 1.00 | 1.00 | 1.00 | 1.00 | 1.00 |

As a result of analyzing bacteria-derived EVs in blood at a family level, a diagnostic model developed using bacteria belonging to the family *Sphingomonadaceae,* the family *Deferribacteraceae,* the family *Weeksellaceae,* the family *Peptococcaceae,* the family *Rhodobacteraceae,* the family *Nocardiaceae,* the family *Neisseriaceae,* the family *Tissierellaceae,* the family *Flavobacteriaceae,* the family *Paraprevotellaceae,* the family *Oxalobacteraceae,* the family *Gemellaceae,* the family *Aerococcaceae,* the family *Leptotrichiaceae,* the family *Rhodocyclaceae,* the family *Williamsiaceae,* and the family *Deinococcaceae* as a biomarker exhibited significant diagnostic performance for Alzheimer's dementia (see Table 5 and FIG. 5).

**[Table 5]**

| | Normal Individual | | Alzheimer's dementia | | t-test | | Training Set | | | Test Set | | |
|---|---|---|---|---|---|---|---|---|---|---|---|---|
| Family | Mean | SD | Mean | SD | p-value | Ratio | AU C | sensiti vity | specifi city | AUC | sensiti vity | spec ificit y |
| Sphingomona daceae | 0.0037 | 0.0028 | 0.0407 | 0.0639 | 0.0003 | 11.03 | 1.00 | 1.00 | 1.00 | 1.00 | 1.00 | 1.00 |
| Deferribactera ceae | 0.0049 | 0.0028 | 0.0109 | 0.008 | 0.0001 | 2.23 | 1.00 | 1.00 | 1.00 | 1.00 | 1.00 | 1.00 |
| [Weeksellace ae] | 0.0094 | 0.0107 | 0.0035 | 0.0030 | 0.0001 | 0.37 | 1.00 | 1.00 | 1.00 | 1.00 | 1.00 | 1.00 |
| Peptococcace ae | 0.0026 | 0.0025 | 0.0052 | 0.0053 | 0.0032 | 2.02 | 1.00 | 1.00 | 1.00 | 1.00 | 1.00 | 1.00 |
| Rhodobactera ceae | 0.0005 | 0.0008 | 0.0019 | 0.0024 | 0.0005 | 3.84 | 1.00 | 1.00 | 1.00 | 1.00 | 1.00 | 1.00 |
| Nocardiaceae | 0.0027 | 0.0030 | 0.0007 | 0.0011 | 0.0000 | 0.26 | 1.00 | 1.00 | 1.00 | 1.00 | 1.00 | 1.00 |
| Neisseriaceae | 0.0021 | 0.0033 | 0.0009 | 0.0013 | 0.0145 | 0.43 | 1.00 | 1.00 | 1.00 | 1.00 | 1.00 | 1.00 |
| [Tissierellace ae] | 0.0015 | 0.0015 | 0.0007 | 0.0008 | 0.0012 | 0.49 | 1.00 | 1.00 | 1.00 | 1.00 | 1.00 | 1.00 |
| Flavobacteria ceae | 0.0011 | 0.0016 | 0.0005 | 0.0006 | 0.0062 | 0.42 | 1.00 | 1.00 | 1.00 | 1.00 | 1.00 | 1.00 |
| [Paraprevotell aceae] | 0.0005 | 0.0007 | 0.0013 | 0.0017 | 0.0059 | 2.53 | 1.00 | 1.00 | 1.00 | 1.00 | 1.00 | 1.00 |
| Oxalobacterac eae | 0.0017 | 0.0028 | 0.0005 | 0.0011 | 0.0042 | 0.30 | 1.00 | 1.00 | 1.00 | 1.00 | 1.00 | 1.00 |
| Gemellaceae | 0.0004 | 0.0009 | 0.0010 | 0.0018 | 0.0432 | 2.72 | 1.00 | 1.00 | 1.00 | 1.00 | 1.00 | 1.00 |
| Aerococcacea e | 0.0004 | 0.0008 | 0.0001 | 0.0003 | 0.0356 | 0.36 | 1.00 | 1.00 | 1.00 | 1.00 | 1.00 | 1.00 |
| Leptotrichiace ae | 0.0002 | 0.0004 | 0.0008 | 0.0014 | 0.0064 | 3.94 | 1.00 | 1.00 | 1.00 | 1.00 | 1.00 | 1.00 |
| Rhodocyclace ae | 0.0042 | 0.0057 | 0.0017 | 0.0017 | 0.0018 | 0.40 | 1.00 | 1.00 | 1.00 | 0.99 | 1.00 | 0.90 |
| Williamsiacea e | 0.0004 | 0.0008 | 0.0013 | 0.0021 | 0.0124 | 2.97 | 1.00 | 1.00 | 1.00 | 0.96 | 0.92 | 1.00 |
| Deinococcace ae | 0.0010 | 0.0019 | 0.0004 | 0.0008 | 0.0184 | 0.38 | 1.00 | 1.00 | 1.00 | 0.95 | 0.92 | 0.90 |

As a result of analyzing bacteria-derived EVs in blood at a genus level, a diagnostic model developed using bacteria belonging to the genus *Sphingomonas,* the genus *Mucispirillum,* the genus *Cloacibacterium,* the genus rc4-4, the genus *Collinsella,* the genus *Rothia,* the genus *Dechloromonas,* the genus *Rhodococcus,* the genus *Neisseria,* the genus *Paracoccus,* the genus *Citrobacter,* the genus *Porphyromonas,* the genus *Anaerococcus,* the genus *Prevotella,* the genus *Tepidimonas,* the genus *Leptotrichia,* the genus *Capnocytophaga,* the genus *Adlercreutzia,* the genus *Williamsia,* and the genus *Deinococcus* as a biomarker exhibited significant diagnostic performance for Alzheimer's dementia (see Table 6 and FIG. 6).

**[Table 6]**

| | Normal Individual | | Alzheimer's dementia | | t-test | | Training Set | | | Test Set | | |
|---|---|---|---|---|---|---|---|---|---|---|---|---|
| Genus | Mean | SD | Mean | SD | p-value | Ratio | AUC | sensiti vity | specifi city | AUC | sensit ivity | specif icity |
| Sphingomo nas | 0.0026 | 0.0024 | 0.0397 | 0.0639 | 0.0003 | 15.50 | 1.00 | 1.00 | 1.00 | 1.00 | 1.00 | 1.00 |
| Mucispirill um | 0.0049 | 0.0028 | 0.0109 | 0.0088 | 0.0001 | 2.23 | 1.00 | 1.00 | 1.00 | 1.00 | 1.00 | 1.00 |
| Cloacibacte rium | 0.0091 | 0.0106 | 0.0033 | 0.0029 | 0.0002 | 0.37 | 1.00 | 1.00 | 1.00 | 1.00 | 1.00 | 1.00 |
| rc4-4 | 0.0026 | 0.0025 | 0.0052 | 0.0053 | 0.0032 | 2.03 | 1.00 | 1.00 | 1.00 | 1.00 | 1.00 | 1.00 |
| Collinsella | 0.0047 | 0.0053 | 0.0016 | 0.0021 | 0.0001 | 0.35 | 1.00 | 1.00 | 1.00 | 1.00 | 1.00 | 1.00 |
| Rothia | 0.0020 | 0.0036 | 0.0009 | 0.0014 | 0.0405 | 0.47 | 1.00 | 1.00 | 1.00 | 1.00 | 1.00 | 1.00 |
| Dechlorom onas | 0.0032 | 0.0043 | 0.0012 | 0.0014 | 0.0012 | 0.37 | 1.00 | 1.00 | 1.00 | 1.00 | 1.00 | 0.90 |
| Rhodococc us | 0.0027 | 0.0030 | 0.0007 | 0.0011 | 0.0000 | 0.24 | 1.00 | 1.00 | 1.00 | 1.00 | 1.00 | 1.00 |
| Neisseria | 0.0015 | 0.0031 | 0.0005 | 0.0007 | 0.0155 | 0.33 | 1.00 | 1.00 | 1.00 | 1.00 | 1.00 | 1.00 |
| Paracoccus | 0.0005 | 0.0008 | 0.0018 | 0.0024 | 0.0005 | 4.08 | 1.00 | 1.00 | 1.00 | 1.00 | 1.00 | 1.00 |
| Citrobacter | 0.0015 | 0.0021 | 0.0004 | 0.0015 | 0.0018 | 0.24 | 1.00 | 1.00 | 1.00 | 1.00 | 1.00 | 1.00 |
| Porphyrom | 0.0005 | 0.0012 | 0.0012 | 0.0018 | 0.0283 | 2.49 | 1.00 | 1.00 | 1.00 | 1.00 | 1.00 | 1.00 |
| onas | | | | | | | | | | | | |
| Anaerococ cus | 0.0010 | 0.0013 | 0.0003 | 0.0007 | 0.0010 | 0.34 | 1.00 | 1.00 | 1.00 | 1.00 | 1.00 | 1.00 |
| [Prevotella] | 0.0003 | 0.0006 | 0.0009 | 0.0012 | 0.0031 | 2.92 | 1.00 | 1.00 | 1.00 | 1.00 | 1.00 | 1.00 |
| Tepidimon as | 0.0002 | 0.0004 | 0.0011 | 0.0015 | 0.0003 | 5.63 | 1.00 | 1.00 | 1.00 | 1.00 | 1.00 | 0.90 |
| Leptotrichi a | 0.0002 | 0.0004 | 0.0007 | 0.0013 | 0.0073 | 4.27 | 1.00 | 1.00 | 1.00 | 1.00 | 1.00 | 1.00 |
| Capnocyto phaga | 0.0006 | 0.0014 | 0.0001 | 0.0003 | 0.0174 | 0.22 | 1.00 | 1.00 | 1.00 | 1.00 | 1.00 | 0.90 |
| Adlercreutz ia | 0.0017 | 0.0016 | 0.0037 | 0.0048 | 0.0094 | 2.15 | 1.00 | 1.00 | 1.00 | 0.99 | 1.00 | 0.90 |
| Williamsia | 0.0004 | 0.0008 | 0.0013 | 0.0021 | 0.0124 | 2.97 | 1.00 | 1.00 | 1.00 | 0.96 | 0.92 | 1.00 |
| Deinococc us | 0.0010 | 0.0019 | 0.0004 | 0.0008 | 0.0184 | 0.38 | 1.00 | 1.00 | 1.00 | 0.95 | 0.92 | 0.90 |

### Example 5. Mild cognitive impairment Diagnostic Model Based on Metagenomic Analysis of Bacteria-Derived EVs Isolated from Blood of Normal Individual and Mild cognitive impairment patient

EVs were isolated from blood samples of 65 mild cognitive impairment patients and 70 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

As a result of analyzing bacteria-derived EVs in blood at a phylum level, a diagnostic model developed using bacteria belonging to the phylum *Fusobacteria,* the phylum *Cyanobacteria,* the phylum SRI, the phylum TM7, the phylum *Thermi,* the phylum *Chloroflexi,* and the phylum *Armatimonadetes* as a biomarker exhibited significant diagnostic performance for mild cognitive impairment (see Table 7 and FIG. 7).

**[Table 7]**

| | Normal Individual | | Mild cognitive impairment | | t-test | | Training Set | | | Test Set | | |
|---|---|---|---|---|---|---|---|---|---|---|---|---|
| Phylum | Mean | SD | Mean | SD | p-value | Ratio | AUC | sensiti vity | specifi city | AUC | sensiti vity | specif icity |
| Fusobacter ia | 0.0019 | 0.0020 | 0.0100 | 0.0093 | 0.0000 | 5.31 | 1.00 | 1.00 | 1.00 | 1.00 | 1.00 | 1.00 |
| Cyanobact eria | 0.0016 | 0.0019 | 0.0007 | 0.0015 | 0.0034 | 0.43 | 1.00 | 1.00 | 1.00 | 1.00 | 1.00 | 1.00 |
| SRI | 0.0024 | 0.0054 | 0.0002 | 0.0005 | 0.0029 | 0.09 | 1.00 | 1.00 | 1.00 | 1.00 | 1.00 | 1.00 |
| TM7 | 0.0007 | 0.0013 | 0.0003 | 0.0004 | 0.0244 | 0.41 | 1.00 | 1.00 | 1.00 | 1.00 | 1.00 | 1.00 |
| [Thermi] | 0.0011 | 0.0019 | 0.0002 | 0.0004 | 0.0005 | 0.18 | 1.00 | 1.00 | 1.00 | 1.00 | 1.00 | 1.00 |
| Chloroflex i | 0.0005 | 0.0012 | 0.0001 | 0.0003 | 0.0275 | 0.27 | 1.00 | 1.00 | 1.00 | 1.00 | 1.00 | 0.92 |
| Armatimo nadetes | 0.0005 | 0.0009 | 0.0000 | 0.0001 | 0.0001 | 0.04 | 1.00 | 1.00 | 1.00 | 1.00 | 1.00 | 1.00 |

As a result of analyzing bacteria-derived EVs in blood at a class level, a diagnostic model developed using bacteria belonging to the class *Betaproteobacteria,* the class *Fusobacteriia,* the class *Chloroplast,* the class TM7-3, the class *Deinococci,* and the class *Fimbriimonadia* as a biomarker exhibited significant diagnostic performance for mild cognitive impairment (see Table 8 and FIG. 8).

**[Table 8]**

| | Normal Individual | | Mild cognitive impairment | | t-test | | Training Set | | | Test Set | | |
|---|---|---|---|---|---|---|---|---|---|---|---|---|
| Class | Mean | SD | Mean | SD | p-value | Ratio | AUC | sensiti vity | specifi city | AUC | sensiti vity | specif icity |
| Betaproteob acteria | 0.0284 | 0.0317 | 0.0141 | 0.0055 | 0.0011 | 0.50 | 1.00 | 1.00 | 1.00 | 1.00 | 1.00 | 1.00 |
| Fusobacterii a | 0.0019 | 0.0020 | 0.0100 | 0.0093 | 0.0000 | 5.31 | 1.00 | 1.00 | 1.00 | 1.00 | 1.00 | 1.00 |
| Chloroplast | 0.0012 | 0.0017 | 0.0006 | 0.0014 | 0.0334 | 0.49 | 1.00 | 1.00 | 1.00 | 1.00 | 1.00 | 1.00 |
| TM7-3 | 0.0007 | 0.0013 | 0.0003 | 0.0004 | 0.0198 | 0.38 | 1.00 | 1.00 | 1.00 | 1.00 | 1.00 | 1.00 |
| Deinococci | 0.0011 | 0.0019 | 0.0002 | 0.0004 | 0.0005 | 0.18 | 1.00 | 1.00 | 1.00 | 1.00 | 1.00 | 1.00 |
| [Fimbriimon adia] | 0.0005 | 0.0009 | 0.0000 | 0.0001 | 0.0001 | 0.04 | 1.00 | 1.00 | 1.00 | 1.00 | 1.00 | 1.00 |

As a result of analyzing bacteria-derived EVs in blood at an order level, a diagnostic model developed using bacteria belonging to the order *Streptophyta* and the order *Rickettsiales* as a biomarker exhibited significant diagnostic performance for mild cognitive impairment (see Table 9 and FIG. 9).

**[Table 9]**

| | Normal Individual | | Mild cognitive impairment | | t-test | | Training Set | | | Test Set | | |
|---|---|---|---|---|---|---|---|---|---|---|---|---|
| Order | Mean | SD | Mean | SD | p-value | Ratio | AUC | sensiti vity | specifi city | AUC | sensiti vity | speci ficity |
| Streptop hyta | 0.0012 | 0.0017 | 0.0006 | 0.0014 | 0.0366 | 0.50 | 1.00 | 1.00 | 1.00 | 1.00 | 1.00 | 1.00 |
| Rickettsi ales | 0.0016 | 0.0037 | 0.0000 | 0.0001 | 0.0026 | 0.02 | 1.00 | 1.00 | 1.00 | 1.00 | 1.00 | 1.00 |

As a result of analyzing bacteria-derived EVs in blood at a family level, a diagnostic model developed using bacteria belonging to the family *Weeksellaceae,* the family *Fusobacteriaceae,* the family *Xanthomonadaceae,* the family *Rhodocyclaceae,* the family *Odoribacteraceae,* the family *Rhodobacteraceae,* the family *Nocardiaceae,* the family *Oxalobacteraceae,* the family *Microbacteriaceae,* the family *Deinococcaceae,* the family *Paenibacillaceae,* the family *Rhizobiaceae,* and the family *Fimbriimonadaceae* as a biomarker exhibited significant diagnostic performance for mild cognitive impairment (see Table 10 and FIG. 10).

**[Table 10]**

| | Normal Individual | | Mild cognitive impairment | | t-test | | Training Set | | | Test Set | | |
|---|---|---|---|---|---|---|---|---|---|---|---|---|
| Family | Mean | SD | Mean | SD | p-value | Rati ο | AUC | sensit ivity | specif icity | AUC | sensit ivity | specif icity |
| [Weeksellaceae] | 0.0094 | 0.0107 | 0.0039 | 0.0031 | 0.0003 | 0.41 | 1.00 | 1.00 | 1.00 | 1.00 | 1.00 | 1.00 |
| | 0.0017 | 0.0019 | 0.0098 | 0.0093 | 0.0000 | 5.85 | 1.00 | 1.00 | 1.00 | 1.00 | 1.00 | 1.00 |
| Xanthomonadac eae | 0.0042 | 0.0082 | 0.0018 | 0.0031 | 0.0364 | 0.42 | 1.00 | 1.00 | 1.00 | 1.00 | 1.00 | 1.00 |
| Rhodocyclaceae | 0.0042 | 0.0057 | 0.0015 | 0.0015 | 0.0007 | 0.36 | 1.00 | 1.00 | 1.00 | 1.00 | 1.00 | 1.00 |
| e] | 0.0006 | 0.0008 | 0.0037 | 0.0031 | 0.0000 | 6.08 | 1.00 | 1.00 | 1.00 | 1.00 | 1.00 | 1.00 |
| Rhodobacteracea e | 0.0005 | 0.0008 | 0.0023 | 0.0049 | 0.0083 | 4.59 | 1.00 | 1.00 | 1.00 | 1.00 | 1.00 | 1.00 |
| Nocardiaceae | 0.0027 | 0.0030 | 0.0009 | 0.0011 | 0.0000 | 0.32 | 1.00 | 1.00 | 1.00 | 1.00 | 0.91 | 1.00 |
| Oxalobacteracea e | 0.0017 | 0.0028 | 0.0003 | 0.0005 | 0.0003 | 0.17 | 1.00 | 1.00 | 1.00 | 1.00 | 1.00 | 1.00 |
| Microbacteriace ae | 0.0004 | 0.0006 | 0.0015 | 0.0016 | 0.0000 | 4.17 | 1.00 | 1.00 | 1.00 | 1.00 | 1.00 | 1.00 |
| Deinococcaceae | 0.0010 | 0.0019 | 0.0002 | 0.0004 | 0.0008 | 0.17 | 1.00 | 1.00 | 1.00 | 1.00 | 1.00 | 1.00 |
| Paenibacillaceae | 0.0002 | 0.0004 | 0.0006 | 0.0014 | 0.0385 | 2.80 | 1.00 | 1.00 | 1.00 | 1.00 | 1.00 | 1.00 |
| Rhizobiaceae | 0.0002 | 0.0003 | 0.0008 | 0.0011 | 0.0001 | 4.53 | 1.00 | 1.00 | 1.00 | 1.00 | 1.00 | 1.00 |
| [Fimbriimonada ceae] | 0.0005 | 0.0009 | 0.0000 | 0.0001 | 0.0001 | 0.04 | 1.00 | 1.00 | 1.00 | 1.00 | 1.00 | 1.00 |

As a result of analyzing bacteria-derived EVs in blood at a genus level, a diagnostic model developed using bacteria belonging to the genus *Cloacibacterium,* the genus *Fusobacterium,* the genus *Lactococcus,* the genus *Stenotrophomonas,* the genus *Dechloromonas,* the genus *Odoribacter,* the genus *Rhodococcus,* the genus *Flavobacterium,* the genus *Deinococcus,* the genus *Paenibacillus,* the genus *Citrobacter,* and the genus *Fimbriimonas* as a biomarker exhibited significant diagnostic performance for mild cognitive impairment (see Table 11 and FIG. 11).

**[Table 11]**

| | Normal Individual | | Mild cognitive impairment | | t-test | | Training Set | | | | Test Set | | | |
|---|---|---|---|---|---|---|---|---|---|---|---|---|---|---|
| Genus | Mean | SD | Mean | SD | value | Ratio | AUC | Accu racy | tivity | specif icity | AUC | Accu racy | sensit ivity | specif icity |
| Cloacibact erium | 0.0091 | 0.0106 | 0.0037 | 0.0031 | 0.0004 | 0.41 | 1.00 | 1.00 | 1.00 | 1.00 | 1.00 | 1.00 | 1.00 | 1.00 |
| ium | 0.0017 | 0.0019 | 0.0098 | 0.0093 | 0.0000 | 5.88 | 1.00 | 1.00 | 1.00 | 1.00 | 1.00 | 1.00 | 1.00 | 1.00 |
| Lactococc us | 0.0025 | 0.0022 | 0.0062 | 0.0038 | 0.0000 | 2.49 | 1.00 | 1.00 | 1.00 | 1.00 | 1.00 | 1.00 | 1.00 | 1.00 |
| omonas | 0.0034 | 0.0074 | 0.0013 | 0.0025 | 0.0367 | 0.37 | 1.00 | 1.00 | 1.00 | 1.00 | 1.00 | 1.00 | 1.00 | 1.00 |
| Dechloro monas | 0.0032 | 0.0043 | 0.0010 | 0.0011 | 0.0003 | 0.30 | 1.00 | 1.00 | 1.00 | 1.00 | 1.00 | 1.00 | 1.00 | 1.00 |
| Odoribacte r | 0.0005 | 0.0007 | 0.0035 | 0.0031 | 0.0000 | 6.81 | 1.00 | 1.00 | 1.00 | 1.00 | 1.00 | 1.00 | 1.00 | 1.00 |
| Rhodococ cus | 0.0027 | 0.0030 | 0.0009 | 0.0011 | 0.0000 | 0.32 | 1.00 | 1.00 | 1.00 | 1.00 | 1.00 | 1.00 | 1.00 | 1.00 |
| Flavobacte rium | 0.0005 | 0.0010 | 0.0014 | 0.0021 | 0.0045 | 2.71 | 1.00 | 1.00 | 1.00 | 1.00 | 1.00 | 1.00 | 1.00 | 1.00 |
| Deinococc us | 0.0010 | 0.0019 | 0.0002 | 0.0004 | 0.0008 | 0.17 | 1.00 | 1.00 | 1.00 | 1.00 | 1.00 | 1.00 | 1.00 | 1.00 |
| Paenibacill us | 0.0002 | 0.0004 | 0.0006 | 0.0014 | 0.0363 | 3.18 | 1.00 | 1.00 | 1.00 | 1.00 | 1.00 | 1.00 | 1.00 | 1.00 |
| Citrobacte r | 0.0015 | 0.0021 | 0.0005 | 0.0010 | 0.0015 | 0.33 | 1.00 | 1.00 | 1.00 | 1.00 | 0.96 | 0.96 | 0.93 | 1.00 |
| Fimbriimo nas | 0.0005 | 0.0009 | 0.0000 | 0.0001 | 0.0001 | 0.04 | 1.00 | 1.00 | 1.00 | 1.00 | 0.96 | 0.92 | 0.86 | 1.00 |

### Example 6. Alzheimer's dementia Diagnostic Model Based on Metagenomic Analysis of Bacteria-Derived EVs Isolated from Blood of Mild cognitive impairment patient and Alzheimer's dementia patient

EVs were isolated from blood samples of 67 Alzheimer's dementia patients and 65 mild cognitive impairment patients, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

As a result of analyzing bacteria-derived EVs in blood at a phylum level, a diagnostic model developed using bacteria belonging to the phylum *Fusobacteria,* the phylum *Deferribacteres,* and the phylum *Armatimonadetes* as a biomarker exhibited significant diagnostic performance for Alzheimer's dementia (see Table 12 and FIG. 12).

**[Table 12]**

| | Mild cognitive impairment | | Alzheimer's dementia | | t-test | | Training Set | | | | Test Set | | | |
|---|---|---|---|---|---|---|---|---|---|---|---|---|---|---|
| Phylum | Mean | SD | Mean | SD | p-value | Ratio | Auc | Acc urac y | sensit ivity | specif icity | Auc | Accu racy | sensit ivity | speci ficity |
| Fusobacte ria | 0.0100 | 0.0093 | 0.0018 | 0.0019 | 0.0000 | 0.18 | 0.82 | 0.73 | 0.69 | 0.78 | 0.83 | 0.77 | 0.75 | 0.80 |
| Deferriba cteres | 0.0040 | 0.0026 | 0.0109 | 0.0088 | 0.0000 | 2.71 | 0.80 | 0.75 | 0.88 | 0.58 | 0.75 | 0.68 | 0.67 | 0.70 |
| Armatim onadetes | 0.0000 | 0.0001 | 0.0003 | 0.0008 | 0.0352 | 15.14 | 0.60 | 0.65 | 0.92 | 0.31 | 0.45 | 0.64 | 0.92 | 0.30 |

As a result of analyzing bacteria-derived EVs in blood at a class level, a diagnostic model developed using bacteria belonging to the class *Fusobacteriia,* the class *Deferribacteres,* and the class *Alphaproteobacteria* as a biomarker exhibited significant diagnostic performance for Alzheimer's dementia (see Table 13 and FIG. 13).

**[Table 13]**

| | Mild cognitive impairment | | Alzheimer's dementia | | t-test | | Training Set | | | | Test Set | | | |
|---|---|---|---|---|---|---|---|---|---|---|---|---|---|---|
| Class | Mean | SD | Mean | SD | p-value | Ratio | AU C | Accu racy | sensit ivity | specif icity | Auc | Accu racy | sensit ivity | specifi city |
| Fusobacter iia | 0.0100 | 0.0093 | 0.0018 | 0.0019 | 0.0000 | 0.18 | 0.82 | 0.73 | 0.69 | 0.78 | 0.83 | 0.77 | 0.75 | 0.80 |
| Deferribac teres | 0.0040 | 0.0026 | 0.0109 | 0.0088 | 0.0000 | 2.71 | 0.80 | 0.75 | 0.88 | 0.58 | 0.75 | 0.68 | 0.67 | 0.70 |
| Alphaprot eo bacteria | 0.0128 | 0.0305 | 0.0470 | 0.0639 | 0.0015 | 3.67 | 0.76 | 0.70 | 0.90 | 0.44 | 0.71 | 0.68 | 0.92 | 0.40 |

As a result of analyzing bacteria-derived EVs in blood at an order level, a diagnostic model developed using bacteria belonging to the order *Methanobacteriales* as a biomarker exhibited significant diagnostic performance for Alzheimer's dementia (see Table 14 and FIG. 14).

**[Table 14]**

| | Mild cognitive impairment | | Alzheimer's dementia | | t-test | | Training Set | | | | Test Set | | | |
|---|---|---|---|---|---|---|---|---|---|---|---|---|---|---|
| Order | Mean | SD | Mean | SD | p-value | Rati ο | AUC | Accuracy | sensitivity | specificity | AUC | Accuracy | sensitivity | specificity |
| Methanob acteriales | 0.0011 | 0.0020 | 0.0005 | 0.0006 | 0.0295 | 0.42 | 0.64 | 0.55 | 0.55 | 0.55 | 0.60 | 0.55 | 0.50 | 0.67 |

As a result of analyzing bacteria-derived EVs in blood at a family level, a diagnostic model developed using bacteria belonging to the family *Microbacteriaceae,* the family *Fusobacteriaceae,* the family *Aerococcaceae,* the family *Bifidobacteriaceae,* the family *Deferribacteraceae,* the family *Sphingomonadaceae,* the family *Flavobacteriaceae,* the family *Rhizobiaceae,* the family *Leptotrichiaceae,* and the family *Micrococcaceae* as a biomarker exhibited significant diagnostic performance for Alzheimer's dementia (see Table 15 and FIG. 15).

**[Table 15]**

| | Mild cognitive impairment | | Alzheimer's dementia | | t-test | | Training Set | | | | Test Set | | | |
|---|---|---|---|---|---|---|---|---|---|---|---|---|---|---|
| Family | Mean | SD | Mean | SD | p-value | Ratio | AUC | Accuracy | sensitivity | specificity | AUC | Accuracy | sensitivity | specificity |
| Microbacteriaceae | 0.0015 | 0.0016 | 0.0002 | 0.0006 | 0.0000 | 0.11 | 0.83 | 0.77 | 0.69 | 0.89 | 0.92 | 0.86 | 0.83 | 0.90 |
| Fusobacteriaceae | 0.0098 | 0.0093 | 0.0010 | 0.0011 | 0.0000 | 0.10 | 0.86 | 0.75 | 0.73 | 0.78 | 0.91 | 0.77 | 0.75 | 0.80 |
| Aerococcaceae | 0.0007 | 0.0010 | 0.0001 | 0.0003 | 0.0000 | 0.19 | 0.68 | 0.62 | 0.60 | 0.64 | 0.80 | 0.77 | 0.75 | 0.80 |
| Bifidobacteriaceae | 0.0210 | 0.0138 | 0.0096 | 0.0057 | 0.0000 | 0.45 | 0.78 | 0.75 | 0.77 | 0.72 | 0.79 | 0.73 | 0.83 | 0.60 |
| Deferribacteraceae | 0.0040 | 0.0026 | 0.0109 | 0.0088 | 0.0000 | 2.71 | 0.80 | 0.75 | 0.88 | 0.58 | 0.75 | 0.68 | 0.67 | 0.70 |
| Sphingomonadaceae | 0.0060 | 0.0265 | 0.0407 | 0.0639 | 0.0011 | 6.78 | 0.83 | 0.79 | 0.94 | 0.58 | 0.74 | 0.73 | 0.92 | 0.50 |
| Flavobacteriaceae | 0.0018 | 0.0022 | 0.0005 | 0.0006 | 0.0001 | 0.26 | 0.70 | 0.61 | 0.63 | 0.58 | 0.72 | 0.73 | 0.58 | 0.90 |
| Rhizobiaceae | 0.0008 | 0.0011 | 0.0002 | 0.0004 | 0.0001 | 0.20 | 0.70 | 0.65 | 0.60 | 0.72 | 0.69 | 0.68 | 0.67 | 0.70 |
| Leptotrichiaceae | 0.0002 | 0.0004 | 0.0008 | 0.0014 | 0.0054 | 4.25 | 0.70 | 0.71 | 0.90 | 0.47 | 0.52 | 0.59 | 0.75 | 0.40 |
| Micrococcaceae | 0.0040 | 0.0067 | 0.0020 | 0.0019 | 0.0304 | 0.49 | 0.69 | 0.67 | 0.79 | 0.50 | 0.46 | 0.41 | 0.42 | 0.40 |

As a result of analyzing bacteria-derived EVs in blood at a genus level, a diagnostic model developed using bacteria belonging to the genus *Fusobacterium,* the genus *Collinsella,* the genus *Sphingomonas,* the genus *Bifidobacterium,* the genus *Mucispirillum,* the genus *Paracoccus,* the genus *Flavobacterium,* the genus *Blautia,* the genus *Tepidimonas,* the genus *Odoribacter,* the genus *Veillonella,* the genus *Porphyromonas,* and the genus *Leptotrichia* as a biomarker exhibited significant diagnostic performance for Alzheimer's dementia (see Table 16 and FIG. 16).

**[Table 16]**

| | Mild cognitive impairment | | Alzheimer's dementia | | t-test | | Training Set | | | | Test Set | | | |
|---|---|---|---|---|---|---|---|---|---|---|---|---|---|---|
| Genus | Mean | SD | Mean | SD | p-value | Ratio | AUC | Accu racy | sensitivity | specificity | AUC | Accuracy | sensitivity | specificity |
| Fusobacterium | 0.0098 | 0.0093 | 0.0010 | 0.0011 | 0.0000 | 0.10 | 0.86 | 0.75 | 0.73 | 0.78 | 0.91 | 0.77 | 0.75 | 0.80 |
| Collinsella | 0.0046 | 0.0040 | 0.0016 | 0.0021 | 0.0000 | 0.35 | 0.76 | 0.73 | 0.73 | 0.72 | 0.88 | 0.73 | 0.75 | 0.70 |
| Sphingomonas | 0.0050 | 0.0262 | 0.0397 | 0.0639 | 0.0011 | 7.88 | 0.86 | 0.83 | 0.92 | 0.72 | 0.78 | 0.73 | 0.92 | 0.50 |
| Bifidobacterium | 0.0210 | 0.0138 | 0.0094 | 0.0057 | 0.0000 | 0.45 | 0.78 | 0.73 | 0.75 | 0.69 | 0.78 | 0.77 | 0.83 | 0.70 |
| Mucispirillum | 0.0040 | 0.0026 | 0.0109 | 0.0088 | 0.0000 | 2.71 | 0.80 | 0.75 | 0.88 | 0.58 | 0.75 | 0.68 | 0.67 | 0.70 |
| Paracoccus | 0.0005 | 0.0009 | 0.0018 | 0.0024 | 0.0006 | 3.88 | 0.69 | 0.69 | 0.90 | 0.42 | 0.75 | 0.73 | 0.83 | 0.60 |
| Flavobacterium | 0.0014 | 0.0021 | 0.0003 | 0.0006 | 0.0004 | 0.23 | 0.66 | 0.67 | 0.75 | 0.56 | 0.74 | 0.59 | 0.67 | 0.50 |
| Blautia | 0.0048 | 0.0039 | 0.0018 | 0.0016 | 0.0000 | 0.37 | 0.78 | 0.74 | 0.75 | 0.72 | 0.73 | 0.64 | 0.67 | 0.60 |
| Tepidimonas | 0.0002 | 0.0003 | 0.0011 | 0.0015 | 0.0003 | 5.86 | 0.75 | 0.69 | 0.88 | 0.44 | 0.65 | 0.59 | 0.67 | 0.50 |
| Odoribacter | 0.0035 | 0.0031 | 0.0017 | 0.0043 | 0.0212 | 0.48 | 0.73 | 0.68 | 0.85 | 0.44 | 0.61 | 0.45 | 0.67 | 0.20 |
| Veillonella | 0.0110 | 0.0240 | 0.0035 | 0.0059 | 0.0235 | 0.32 | 0.69 | 0.67 | 0.79 | 0.50 | 0.60 | 0.50 | 0.67 | 0.30 |
| Porphyromonas | 0.0006 | 0.0009 | 0.0012 | 0.0018 | 0.0406 | 2.09 | 0.59 | 0.65 | 0.90 | 0.33 | 0.59 | 0.55 | 0.75 | 0.30 |
| Leptotrichia | 0.0002 | 0.0004 | 0.0007 | 0.0013 | 0.0053 | 4.85 | 0.69 | 0.69 | 0.88 | 0.44 | 0.56 | 0.64 | 0.83 | 0.40 |

The above description of the present invention is provided only for illustrative purposes, and it will be understood by one of ordinary skill in the art to which the present invention pertains that the invention may be embodied in various modified forms without departing from the spirit or essential characteristics thereof. Thus, the embodiments described herein should be considered in an illustrative sense only and not for the purpose of limitation.

### [industrial Applicability]

The method of providing information for diagnosing Alzheimer's dementia through a bacterial metagenomic analysis according to the present invention may be used for predicting the risk of Alzheimer's dementia and mild cognitive impairment onset and diagnosing Alzheimer's dementia and mild cognitive impairment by performing a bacterial metagenomic analysis using normal individual-derived and subject-derived samples to analyze an increase or decrease in the content of specific bacteria-derived extracellular vesicles.

## Claims

1. A method of providing information for diagnosing Alzheimer's dementia, the method comprising:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

2. The method of claim 1, wherein the normal individual and subject samples are blood, and
wherein process (c) comprises comparing an increase or decrease in content of extracellular vesicles derived from one or more bacteria selected from the group consisting of the phylum *Deferribacteres,* the phylum SRI, the phylum *Synergistetes,* and the phylum *Thermi*;
extracellular vesicles derived from one or more bacteria selected from the group consisting of the class *Alphaproteobacteria,* the class *Flavobacteriia,* the class *Deferribacteres,* and the class *Deinococci;*
extracellular vesicles derived from bacteria of the order *Rickettsiales,*
extracellular vesicles derived from one or more bacteria selected from the group consisting of the family *Sphingomonadaceae,* the family *Deferribacteraceae,* the family *Weeksellaceae,* the family *Peptococcaceae,* the family *Rhodobacteraceae,* the family *Nocardiaceae,* the family *Neisseriaceae,* the family *Tissierellaceae,* the family *Flavobacteriaceae,* the family *Paraprevotellaceae,* the family *Oxalobacteraceae,* the family *Gemellaceae,* the family *Aerococcaceae,* the family *Leptotrichiaceae,* the family *Rhodocyclaceae,* the family *Williamsiaceae,* and the family *Deinococcaceae;* or
extracellular vesicles derived from one or more bacteria selected from the group consisting of the genus *Sphingomonas,* the genus *Mucispirillum,* the genus *Cloacibacterium,* the genus rc4-4, the genus *Collinsella,* the genus *Rothia,* the genus *Dechloromonas,* the genus *Rhodococcus,* the genus *Neisseria,* the genus *Paracoccus,* the genus *Citrobacter,* the genus *Porphyromonas,* the genus *Anaerococcus,* the genus *Prevotella,* the genus *Tepidimonas,* the genus *Leptotrichia,* the genus *Capnocytophaga,* the genus *Adlercreutzia,* the genus *Williamsia,* and the genus *Deinococcus.*

3. The method of claim 2, wherein process (c), in comparison with the normal individual-derived sample, an increase in the content of the following is diagnosed as Alzheimer's dementia:
extracellular vesicles derived from bacteria of the phylum *Deferribacteres,*
extracellular vesicles derived from one or more bacteria selected from the group consisting of the class *Alphaproteobacteria* and the class *Deferribacteres,*
extracellular vesicles derived from one or more bacteria selected from the group consisting of the family the family *Deferribacteraceae,* the family *Peptococcaceae,* the family *Rhodobacteraceae,* the family *Paraprevotellaceae,* the family *Gemellaceae,* the family *Leptotrichiaceae,* and the family *Williamsiaceae,* or
extracellular vesicles derived from one or more bacteria selected from the group consisting of the genus *Sphingomonas,* the genus *Mucispirillum,* the genus rc4-4, the genus *Paracoccus,* the genus *Porphyromonas,* the genus *Prevotella,* the genus *Tepidimonas,* the genus *Leptotrichia,* the genus *Adlercreutzia,* the genus *Williamsia.*

4. The method of claim 2, wherein process (c), in comparison with the normal individual-derived sample, a decrease in the content of the following is diagnosed as Alzheimer's dementia:
extracellular vesicles derived from one or more bacteria selected from the group consisting of the phylum SRI, the phylum *Synergistetes,* and the phylum *Thermi,*
extracellular vesicles derived from one or more bacteria selected from the group consisting of the class *Flavobacteriia* and the class *Deinococci,*
extracellular vesicles derived from bacteria of the order *Rickettsiales,*
extracellular vesicles derived from one or more bacteria selected from the group consisting of the family *Weeksellaceae,* the family *Nocardiaceae,* the family *Neisseriaceae,* the family *Tissierellaceae,* the family *Flavobacteriaceae,* the family *Oxalobacteraceae,* the family *Aerococcaceae,* the family *Rhodocyclaceae,* the family *Deinococcaceae,*
extracellular vesicles derived from one or more bacteria selected from the group consisting of the genus *Cloacibacterium,* the genus *Collinsella,* the genus *Rothia,* the genus *Dechloromonas,* the genus *Rhodococcus,* the genus *Neisseria,* the genus *Citrobacter,* the genus *Anaerococcus,* the genus *Capnocytophaga,* the genus *Deinococcus.*

5. The method of claim 2, wherein the blood is whole blood, serum, plasma, or blood mononuclear cells.

6. A method of diagnosing Alzheimer's dementia, the method comprising:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

7. A method of providing information for diagnosing mild cognitive impairment, the method comprising:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

8. The method of claim 7, wherein the normal individual and subject samples are blood, and
wherein process (c) comprises comparing an increase or decrease in content of extracellular vesicles derived from one or more bacteria selected from the group consisting of the phylum *Fusobacteria,* the phylum *Cyanobacteria,* the phylum SRI, the phylum TM7, the phylum *Thermi,* the phylum *Chloroflexi,* and the phylum *Armatimonadetes;*
extracellular vesicles derived from one or more bacteria selected from the group consisting of the class *Betaproteobacteria,* the class *Fusobacteriia,* the class *Chloroplast,* the class TM7-3, the class *Deinococci,* and the class *Fimbriimonadia;*
extracellular vesicles derived from one or more bacteria selected from the group consisting of the order *Streptophyta* and the order *Rickettsiales;*
extracellular vesicles derived from one or more bacteria selected from the group consisting of the family *Weeksellaceae,* the family *Fusobacteriaceae,* the family *Xanthomonadaceae,* the family *Rhodocyclaceae,* the family *Odoribacteraceae,* the family *Rhodobacteraceae,* the family *Nocardiaceae,* the family *Oxalobacteraceae,* the family *Microbacteriaceae,* the family *Deinococcaceae,* the family *Paenibacillaceae,* the family *Rhizobiaceae,* and the family *Fimbriimonadaceae;* or
extracellular vesicles derived from one or more bacteria selected from the group consisting of the genus *Cloacibacterium,* the genus *Fusobacterium,* the genus *Lactococcus,* the genus *Stenotrophomonas,* the genus *Dechloromonas,* the genus *Odoribacter,* the genus *Rhodococcus,* the genus *Flavobacterium,* the genus *Deinococcus,* the genus *Paenibacillus,* the genus *Citrobacter,* and the genus *Fimbriimonas.*

9. The method of claim 8, wherein process (c), in comparison with the normal individual-derived sample, an increase in the content of the following is diagnosed as mild cognitive impairment:
extracellular vesicles derived from bacteria of the phylum *Fusobacteria,*
extracellular vesicles derived from bacteria of the class *Fusobacteriia,*
extracellular vesicles derived from one or more bacteria selected from the group consisting of the family *Fusobacteriaceae,* the family *Odoribacteraceae,* the family *Rhodobacteraceae,* the family *Microbacteriaceae,* the family *Paenibacillaceae,* and the family *Rhizobiaceae,* or
extracellular vesicles derived from one or more bacteria selected from the group consisting of the genus *Fusobacterium,* the genus *Lactococcus,* the genus *Odoribacter,* the genus *Flavobacterium,* and the genus *Paenibacillus.*

10. The method of claim 8, wherein process (c), in comparison with the normal individual-derived sample, a decrease in the content of the following is diagnosed as mild cognitive impairment:
extracellular vesicles derived from one or more bacteria selected from the group consisting of the phylum *Cyanobacteria,* the phylum SRI, the phylum TM7, the phylum *Thermi,* the phylum *Chloroflexi,* and the phylum *Armatimonadetes,*
extracellular vesicles derived from one or more bacteria selected from the group consisting of the class *Betaproteobacteria,* the class *Chloroplast,* the class TM7-3, the class *Deinococci,* and the class *Fimbriimonadia,*
extracellular vesicles derived from one or more bacteria selected from the group consisting of the order *Streptophyta* and the order *Rickettsiales,*
extracellular vesicles derived from one or more bacteria selected from the group consisting of the family *Weeksellaceae,* the family *Xanthomonadaceae,* the family *Rhodocyclaceae,* the family *Nocardiaceae,* the family *Oxalobacteraceae,* the family *Deinococcaceae,* and the family *Fimbriimonadaceae,* or
extracellular vesicles derived from one or more bacteria selected from the group consisting of the genus *Cloacibacterium,* the genus *Stenotrophomonas,* the genus *Dechloromonas,* the genus *Rhodococcus,* the genus *Deinococcus,* the genus *Citrobacter,* and the genus *Fimbriimonas.*

11. The method of claim 8, wherein the blood is whole blood, serum, plasma, or blood mononuclear cells.

12. A method of diagnosing mild cognitive impairment, the method comprising:
(a) extracting DNAs from extracellular vesicles isolated from normal individual and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a normal individual-derived sample through sequencing of a product of the PCR.

13. A method of providing information for diagnosing Alzheimer's dementia, the method comprising:
(a) extracting DNAs from extracellular vesicles isolated from mild cognitive impairment patient and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a mild cognitive impairment patient-derived sample through sequencing of a product of the PCR.

14. The method of claim 13, wherein the mild cognitive impairment patient and subject samples are blood, and
wherein process (c) comprises comparing an increase or decrease in content of extracellular vesicles derived from one or more bacteria selected from the group consisting of the phylum *Fusobacteria,* the phylum *Deferribacteres,* and the phylum *Armatimonadetes;*
extracellular vesicles derived from one or more bacteria selected from the group consisting of the class *Fusobacteriia,* the class *Deferribacteres,* and the class *Alphaproteobacteria;*
extracellular vesicles derived from bacteria of the order *Methanobacteriales;* extracellular vesicles derived from one or more bacteria selected from the group consisting of the family *Microbacteriaceae,* the family *Fusobacteriaceae,* the family *Aerococcaceae,* the family *Bifidobacteriaceae,* the family *Deferribacteraceae,* the family the family *Flavobacteriaceae,* the family *Rhizobiaceae,* the family *Leptotrichiaceae,* and the family *Micrococcaceae;* or
extracellular vesicles derived from one or more bacteria selected from the group consisting of the genus *Fusobacterium,* the genus *Collinsella,* the genus *Sphingomonas,* the genus *Bifidobacterium,* the genus *Mucispirillum,* the genus *Paracoccus,* the genus *Flavobacterium,* the genus *Blautia,* the genus *Tepidimonas,* the genus *Odoribacter,* the genus *Veillonella,* the genus *Porphyromonas,* and the genus *Leptotrichia.*

15. The method of claim 14, wherein process (c), in comparison with the mild cognitive impairment patient-derived sample, an increase in the content of the following is diagnosed as Alzheimer's dementia:
extracellular vesicles derived from one or more bacteria selected from the group consisting of the phylum *Deferribacteres* and the phylum *Armatimonadetes,*
extracellular vesicles derived from one or more bacteria selected from the group consisting of the class *Deferribacteres* and the class *Alphaproteobacteria,*
extracellular vesicles derived from one or more bacteria selected from the group consisting of the family *Deferribacteraceae,* the family and the family *Leptotrichiaceae,* or
extracellular vesicles derived from one or more bacteria selected from the group consisting of the genus *Sphingomonas,* the genus *Mucispirillum,* the genus *Paracoccus,* the genus *Tepidimonas,* the genus *Porphyromonas,* and the genus *Leptotrichia.*

16. The method of claim 14, wherein process (c), in comparison with the mild cognitive impairment patient-derived sample, a decrease in the content of the following is diagnosed as Alzheimer's dementia:
extracellular vesicles derived from bacteria of the phylum *Fusobacteria,*
extracellular vesicles derived from bacteria of the class *Fusobacteriia,*
extracellular vesicles derived from bacteria of the order *Methanobacteriales,*
extracellular vesicles derived from one or more bacteria selected from the group consisting of the family *Microbacteriaceae,* the family *Fusobacteriaceae,* the family *Aerococcaceae,* the family *Bifidobacteriaceae,* the family *Flavobacteriaceae,* the family *Rhizobiaceae,* and the family *Micrococcaceae,* or
extracellular vesicles derived from one or more bacteria selected from the group consisting of the genus *Fusobacterium,* the genus *Collinsella,* the genus *Bifidobacterium,* the genus *Flavobacterium,* the genus *Blautia,* the genus *Odoribacter,* and the genus *Veillonella.*

17. The method of claim 14, wherein the blood is whole blood, serum, plasma, or blood mononuclear cells.

18. A method of diagnosing Alzheimer's dementia, the method comprising:
(a) extracting DNAs from extracellular vesicles isolated from mild cognitive impairment patient and subject samples;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject-derived sample with that of a mild cognitive impairment patient-derived sample through sequencing of a product of the PCR.


<110>    MD Healthcare Inc.
         Ewha University - Industry Collaboration Foundation

<120>    Method for diagnosis of Alzheimer dementia using analysis of
         bacteria metagenome

<130>    MPCT18-052

<150>    KR 10-2017-0133372
<151>    2017-10-13

<150>    KR 10-2018-0060753
<151>    2018-05-28

<160>    2

<170>    KoPatentIn 3.0

<210>    1
<211>    50
<212>    DNA
<213>    Artificial Sequence

<220>
<223>    Forward primer for 16s rDNA


<400>    1
tcgtcggcag cgtcagatgt gtataagaga cagcctacgg gnggcwgcag                50


<210>    2
<211>    55
<212>    DNA
<213>    Artificial Sequence

<220>
<223>    Reverse primer for 16s rDNA


<400>    2
gtctcgtggg ctcggagatg tgtataagag acaggactac hvgggtatct aatcc          55